(19) Europäisches Patentamt
European Patent Office
Office européen des brevets

(11) EP 4 338 662 B1

(12) EUROPEAN PATENT SPECIFICATION

(45) Date of publication and mention of the grant of the patent:
**09.07.2025 Bulletin 2025/28**

(21) Application number: **23215181.1**

(22) Date of filing: **26.09.2019**

(51) International Patent Classification (IPC):
***A61N 1/36*** *(2006.01)* ***A61N 1/18*** *(2006.01)*
***A61N 1/04*** *(2006.01)* ***A61B 5/11*** *(2006.01)*
***A61B 5/24*** *(2021.01)* ***A61B 5/00*** *(2006.01)*

(52) Cooperative Patent Classification (CPC):
**A61B 5/1101; A61B 5/24; A61B 5/4041; A61B 5/6847; A61B 5/6868; A61B 5/7207; A61B 5/7257; A61N 1/0456; A61N 1/3603;**
A61N 1/36003; A61N 1/36025; A61N 1/36031

(54) **PREDICTIVE THERAPY NEUROSTIMULATION SYSTEMS**

NEUROSTIMULATIONSSYSTEME FÜR PRÄDIKTIVE THERAPIE

SYSTÈMES DE NEUROSTIMULATION THÉRAPEUTIQUE PRÉDICTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2018 US 201862736968 P**

(43) Date of publication of application:
**20.03.2024 Bulletin 2024/12**

(60) Divisional application:
**25179668.6**

(62) Document number(s) of the earlier application(s) in accordance with Art. 76 EPC:
**19866694.3 / 3 856 330**

(73) Proprietor: **Cala Health, Inc.**
**San Mateo, CA 94404 (US)**

(72) Inventors:
- **ROSS, Erika Kristine**
  **Burlingame, 94010 (US)**
- **SHIN, Sooyoon**
  **Burlingame, 94010 (US)**
- **HOUSTON, Brady**
  **Burlingame, 94010 (US)**
- **BALBAKY, Sami**
  **Burlingame, 94010 (US)**
- **HAMNER, Samuel Richard**
  **Burlingame, 94010 (US)**
- **ROSENBLUTH, Kathryn H.**
  **Burlingame, 94010 (US)**
- **YU, Jai Y.**
  **Burlingame, 94010 (US)**

(74) Representative: **J A Kemp LLP**
**80 Turnmill Street**
**London EC1M 5QU (GB)**

(56) References cited:
**WO-A1-2014/089549**
**WO-A1-2018/028170**
**WO-A2-2017/132067**
**US-A1- 2014 336 722**
**US-A1- 2016 198 998**
**US-A1- 2018 264 263**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

BACKGROUND

### Field of the Invention

Embodiments of the invention relate generally to devices for stimulating nerves, and more specifically relate to devices for electrically stimulating peripheral nerve(s) to treat various disorders, as well as signal processing systems and methods for enhancing diagnostic and therapeutic protocols relating to the same.

### Description of the Related Art

A wide variety of modalities can be utilized to neuromodulate peripheral nerves. For example, electrical energy can be delivered transcutaneously via electrodes on the skin surface with neurostimulation systems to stimulate peripheral nerves, such as the median, radial, and/or ulnar nerves in the upper extremities; the tibial, saphenous, and/or peroneal nerve in the lower extremities; or the auricular vagus, tragus, trigeminal or cranial nerves on the head or ear, as non-limiting examples. Stimulation of these nerves has been shown to provide therapeutic benefit across a variety of diseases, including but not limited to movement disorders (including but not limited to essential tremor, Parkinson's tremor, orthostatic tremor, and multiple sclerosis), urological disorders, gastrointestinal disorders, cardiac diseases, and inflammatory diseases, mood disorders (including but not limited to depression, bipolar disorder, dysthymia, and anxiety disorder), pain syndromes (including but not limited to migraines and other headaches, trigeminal neuralgia, fibromyalgia, complex regional pain syndrome), among others. A number of conditions, such as tremors, can be treated through some form of transcutaneous, percutaneous, or other implanted forms of peripheral nerve stimulation. Wearable systems with compact, ergonomic form factors are needed to enhance efficacy, compliance, and comfort with using the devices.

WO 2017/132067A discloses systems and methods which include a wearable device with an electrically conductive skin interface that excites the underlying nerves from a transcutaneous surface stimulator. The device may be sized for a range of user sizes with stimulation electrodes positioned to target the appropriate nerves, such as the saphenous and/or posterior tibial nerves. Transcutaneous afferent stimulation of one, two, or more peripheral nerves can modulate a brain or spinal pathway associated with bladder function.

WO 2014/089549A discloses a system and method for monitoring nerve activity in a subject. The system includes a plurality of electrodes placed in proximity to skin of the subject, an amplifier electrically connected to the electrodes and configured to generate a plurality of amplified signals corresponding to electrical signals received from the subject through the electrodes, and a signal processor. The signal processor applies a high-pass filter to the amplified signals to generate filtered signals from the amplified signals, identifies autonomic nerve activity in the plurality of filtered signals; and generates an output signal corresponding to the filtered signals. The high-pass filter attenuates a plurality of the amplified signals having frequencies that correspond to heart muscle activity during a heartbeat.

US 2014/336722A discloses a method and neuroprosthetic device for monitoring and suppression of pathological tremors in a user through the neurostimulation of the afferent pathways to the brain, which comprises wearable elements placed over the parts of the body affected by tremor, wherein said wearable elements have sensors selected from bioelectric sensors generating a bioelectrical signal characterization of tremor, biomechanical sensors generating a biomechanical signal characterization of tremor and a combination thereof, a programmable electronic device comprised of a control, acquisition and processing module of the characterization signals, and a signal generator for the afferent stimulation based on the bioelectrical, biomechanical or combination of both signal characterization and stimulation electrodes which transmit the neuromodulation signals to the afferent pathways projecting into the central nervous system to modulate the activity of the neural structures responsible for tremor generation.

WO 2018/028170A discloses a method for encoding a multi-channel signal and an encoder, the encoding method comprising: obtaining a multi-channel signal of a current frame; determining an initial multi-channel parameter of the current frame; determining a difference parameter according to the initial multi-channel parameter of the current frame and multi-channel parameters of the former K frames of the current frame, the difference parameter being used for representing a difference between the initial multi-channel parameter of the current frame and the multi-channel parameters of the former K frames, wherein K is an integer greater than or equal to 1; determining a multi-channel parameter of the current frame according to the difference parameter and a feature parameter of the current frame; and encoding the multi-channel signal according to the multi-channel parameter of the current frame. The present invention can better ensure the accuracy of inter-channel information of a multi-channel signal.

US 2018/264263A discloses a peripheral nerve stimulator which can be used to stimulate a peripheral nerve to treat essential tremor, Parkinson tremor, and other forms of tremor. The peripheral nerve stimulator can be either a noninvasive surface stimulator or an implanted stimulator. Stimulation can be electrical, mechanical, or chemical. Stimulation can be delivered using either an open loop system or a closed loop system with feedback.

US 2016/198998A discloses a system for obtaining and analyzing data for overall joint motion from a plurality of joints of a subject experiencing a movement disorder involves a plurality of kinematic sensors configured to be placed on a body of a subject experiencing a movement disorder proximal a plurality of joints of the subject. The kinematic sensors are selected to measure overall joint motion with sufficient degrees of freedom for individual joints so that data collected by the sensors can be deconstructed into multiple degrees of freedom for individual joints and analyzed to provide amplitude of the movements caused by the movement disorder and/or relative contributions from and/or directional bias for each muscle group that may be implicated in the movement of each joint. Such a system permits methods for determining a treatment regimen for treating the movement disorder, whereby the treatment regimen is based on the amplitude of the movements and/or the relative contribution and/or directional bias of each muscle group to the movements caused by the movement disorder.

## SUMMARY

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

Disclosed herein is a neuromodulation device.

Also disclosed herein are systems for predicting a clinical score.

Further disclosed herein are systems for predicting a response to therapy or lack thereof, according to any one or more of the embodiments described in the disclosure.

Disclosed herein is a wearable neurostimulation device for transcutaneously stimulating one or more peripheral nerves of a user. The device can include one or more electrodes configured to generate electric stimulation signals. The device can further include one or more sensors configured to detect motion signals, wherein the one or more sensors are operably connected to the wearable neurostimulation device; The device can also include one or more hardware processors. The one or more hardware processors can receive raw signals in time domain from the one or more sensors. The one or more hardware processors can separate the raw signals into a plurality of frames. In some instances, for each of the plurality of frames, the one or more hardware processors can transform the raw signals into a frequency domain. Further, the one or or more hardware processors can calculate a first energy in a first frequency band of the transformed signal for a respective frame. The one or more hardware processors can also calculate a second energy in a second frequency band of the transformed signal for the respective frame, wherein the second frequency band includes a first frequency corresponding to a tremor. The one or more hardware processors can determine motion artifact in the respective frame based on a comparison of the first energy with the second energy. The one or more hardware processors can combine frames based on the determination of motion artifact for each of the frames. Furthermore, the one or more hardware processors can extract features from the combined frames in a time domain or frequency domain. The one or more hardware processors can determine rules based on the extracted features. In some instances, the one or more hardware processor can determine neurostimulation therapy outcomes based on an application of the determined rules on operational data.

In some instances, the sensors are operably attached to the wearable neurostimulation device.

In some instances, the raw signals relate to tremor activity of the user.

In some instances, the wearable neurostimulation device can include one or more end effectors that can generate stimulation signals other than electric stimulation signals.

In some instances, the stimulation signals other than electric stimulation signals are vibrational stimulation signals.

In some instances, the sensors include an IMU. The IMU can include one or more of a gyroscope, accelerometer, and magnetometer.

In some instances, the second frequency band is between about 4 Hz and about 12 Hz.

In some instances, the second frequency band is between about 3 Hz and about 8 Hz.

In some instances, the features can include any one or more of the following: amplitude, bandwidth, area under the curve (e.g., power), energy in frequency bins, peak frequency, or ratio between frequency bands.

In some instances, the features include one or more of kinematic features. The kinematic features can include regularity, amplitude and shape of the signal.

In some instances, the features can include any one or more of the following: amplitude or power spectral density ("PSD") at peak tremor frequency, summed amplitude or PSD in a band that is about 2.75 Hz wide surrounding the peak tremor frequency, summed amplitude or PSD in a band between about 4 to about 12 Hz, or summed amplitude or PSD in a band surrounding the peak tremor frequency selected only from the pre-stimulation spectra.

In some instances, the features include time domain features. In some instances, the features include frequency domain features. In some instances, the features include a combination of time domain and frequency domain features.

In some instances, the features include any one or more of the following: at least one of approximate entropy, displacement, curve fitting, functional PCA, filtering, mean, median, or range in time domain.

Disclosed herein is wearable device for transcutaneously modulating one or more peripheral nerves of a user. The device can include one or more end effectors configured to generate stimulation signals. The device can further include one or more sensors configured to detect motion signals, wherein the one or more sensors are operably connected to the wearable device. The device can include one or more hardware processors. The one or more hardware processors can receive raw signals in time domain from the one or more sensors. The one or more hardware processors can separate the raw signals into a plurality of frames. In some instances, for each of the plurality of frames, the one or more hardware processors can execute one or more of the following operations: transform the raw signals into a frequency domain; calculate a first energy in a first frequency band of the transformed signal for a respective frame; calculate a second energy in a second frequency band of the transformed signal for the respective frame, wherein the second frequency band includes a first frequency corresponding to a tremor; determine motion artifact in the respective frame based on a comparison of the first energy with the second energy; combine frames based on the determination of motion artifact for each of the frames; extract features from the combined frames in a time domain or frequency domain; determine rules based on the extracted features; and determine one or more of clinical scores and neurostimulation therapy outcomes based on an application of the determined rules on operational data.

In some instances, the one or more hardware processors can determine a first calibration frequency for a first stimulation therapy during a first activity, and a second, different calibration frequency for a second stimulation therapy during a second, different activity. In some instances, the first calibration frequency is within about 3 Hz of the second calibration frequency.

BRIEF DESCRIPTION OF THE DRAWINGS

Figure 1A illustrates a block diagram of an example neuromodulation (e.g., neurostimulation) device.
Figure 1B illustrates a block diagram of an embodiment of a controller that can be implemented with the hardware components described with respect to Figure 1A.
Figure 1C schematically illustrates an embodiment of a neuromodulation device and base station.
Figure 2 illustrates a block diagram of an embodiment of a controller that can be implemented with the hardware components described with respect to Figure 1A or 1B.
Figure 3 illustrates a flow chart of an embodiment of a process for calibration of neurostimulation device.
Figure 3A illustrates a flow chart of a non-limiting embodiment of a process for performing windowed calibration.
Figure 3B is a graph that illustrates that in some cases, a patient's tremor frequency can vary across different tasks.
Figures 4 and 4A illustrate flow charts of embodiments of a process for collecting data from IMU and processing the collected data.
Figure 5A illustrates a flowchart of an embodiment of a process for generation of rules to determine neurostimulation therapy outcomes.
Figure 5B illustrates an example combined spectrum including example extracted features.
Fig. 6A schematically illustrates a wearable neuromodulation device.
Fig. 6B illustrates an embodiment of stimulation waveforms.
Fig. 6C illustrates stimulation assessment at different time points.
Fig. 6D illustrates features extracted from a power spectral density plot.
Figs. 7A - 7D illustrate reduction in sensor measured tremor kinematics with respect to neuromodulation therapy.
Figs. 8A-8D illustrate correlation graphs between mean peak tremor power and clinical visual ratings.
Figs. 9A-9D illustrate that sensor measured kinematics can predict clinical ratings across tasks.
Fig. 10 is a graph illustrating a correlation between motion data (e.g., accelerometry) and clinical scale
Fig. 11 demonstrates that prediction of patient response can improve with data collected over a longer period of time.
Fig. 12 further demonstrates how patient response on first day / first week / first month of therapy predicts response over all sessions based on average outcome during a study.
Fig. 13 illustrates that the efficacy of neuromodulation therapy is high for both patients on and off medications (e.g., tremor medications) at the time of neuromodulation therapy.
Fig. 14 is a graph illustrating that patient measured therapy outcome and sensor measured kinematic improvement were correlated.
Figs. 15A-15D illustrate examples of endpoints for sensor measures, according to some embodiments.

DETAILED DESCRIPTION

Disclosed herein are devices configured for providing neuromodulation (e.g., neurostimulation). The neuromodulation (e.g., neurostimulation) devices provided herein may be configured to stimulate peripheral nerves of a user. The neuromodulation (e.g., neurostimulation) devices may be configured to transcutaneously transmit one or more neuromodulation (e.g., neurostimulation) signals across the skin of the user. In many embodiments, the neuromodulation (e.g., neurostimulation) devices are wearable devices configured to be worn by a user. The user may be a human, another mammal, or other animal user. The neuromodulation (e.g., neurostimulation) system could also include signal processing systems and methods for enhancing diagnostic and therapeutic protocols relating to the same. In some embodiments, the neuromodulation (e.g., neurostimulation) device is configured to be wearable on an upper extremity of a user (e.g., a wrist, forearm, arm, and/or finger(s) of a user). In some embodiments, the device is configured to be wearable on a lower extremity (e.g., ankle, calf, knee, thigh, foot, and/or toes) of a user. In some embodiments, the device is configured to be wearable on the head or neck (e.g., forehead, ear, neck, nose, and/or tongue). In several embodiments, dampening or blocking of nerve impulses and/or neurotransmitters are provided. In some embodiments, nerve impulses and/or neurotransmitters are enhanced. In some embodiments, the device is configured to be wearable on or proximate an ear of a user, including but not limited to auricular neuromodulation (e.g., neurostimulation) of the auricular branch of the vagus nerve, for example. The device could be unilateral or bilateral, including a single device or multiple devices connected with wires or wirelessly.

Systems with compact, ergonomic form factors are needed to enhance efficacy, compliance, and/or comfort when using non-invasive or wearable neuromodulation devices. In several embodiments, neuromodulation systems and methods are provided that enhance or inhibit nerve impulses and/or neurotransmission, and/or modulate excitability of nerves, neurons, neural circuitry, and/or other neuroanatomy that affects activation of nerves and/or neurons. For example, neuromodulation (e.g., neurostimulation) can include one or more of the following effects on neural tissue: depolarizing the neurons such that the neurons fire action potentials; hyperpolarizing the neurons to inhibit action potentials; depleting neuron ion stores to inhibit firing action potentials; altering with proprioceptive input; influencing muscle contractions; affecting changes in neurotransmitter release or uptake; and/or inhibiting firing.

In some embodiments, wearable systems and methods as disclosed herein can advantageously be used to identify whether a treatment is effective in significantly reducing or preventing a medical condition, including but not limited to tremor severity. Wearable sensors can advantageously monitor, characterize, and aid in the clinical management of hand tremor as well as other medical conditions including those disclosed elsewhere herein. Not to be limited by theory, clinical ratings of medical conditions, e.g., tremor severity can correlate with simultaneous measurements of wrist motion using inertial measurement units (IMUs). For example, tremor features extracted from IMUs at the wrist can provide characteristic information about tremor phenotypes that may be leveraged to improve diagnosis, prognosis, and/or therapeutic outcomes. Kinematic measures can correlate with tremor severity, and machine learning algorithms incorporated in neuromodulation systems and methods as disclosed for example herein can predict the visual rating of tremor severity.

## Neuromodulation Device

Figure 1A illustrates a block diagram of an example neuromodulation (e.g., neurostimulation) device 100. The device 100 includes multiple hardware components which are capable of, or programmed to provide therapy across the skin of the user. As illustrated in Figure 1A, some of these hardware components may be optional as indicated by dashed blocks. In some instances, the device 100 may only include the hardware components that are required for stimulation therapy. The hardware components are described in more detail below.

The device 100 can include two or more effectors, e.g. electrodes 102 for providing neurostimulation signals. In some instances, the device 100 is configured for transcutaneous use only and does not include any percutaneous or implantable components. In some embodiments, the electrodes can be dry electrodes. In some embodiments, water or gel can be applied to the dry electrode or skin to improve conductance. In some embodiments, the electrodes do not include any hydrogel material, adhesive, or the like.

The device 100 can further include stimulation circuitry 104 for generating signals that are applied through the electrode(s) 102. The signals can vary in frequency, phase, timing, amplitude, or offsets. The device 100 can also include power electronics 106 for providing power to the hardware components. For example, the power electronics 106 can include a battery.

The device 100 can include one or more hardware processors 108. The hardware processors 108 can include microcontrollers, digital signal processors, application specific integrated circuit (ASIC), a field programmable gate array (FPGA) or other programmable logic device, discrete gate or transistor logic, discrete hardware components, or any combination thereof designed to perform the functions described herein. In an embodiment, all of the processing discussed herein is performed by the hardware processor(s) 108. The memory 110 can store data specific to patient and rules as discussed below.

In the illustrated figure, the device 100 can include one or more sensors 112. As shown in the figure, the sensor(s) 112 may be optional. Sensors could include, for example, biomechanical sensors configured to, for example, measure motion, and/or bioelectrical sensors (e.g., EMG, EEG, and/or nerve conduction sensors). Sensors can include, for example, cardiac activity sensors (e.g., ECG, PPG), skin conductance sensors (e.g., galvanic skin response, electrodermal activity), and motion sensors (e.g., accelerometers, gyroscopes). The one or more sensors 102 may include an inertial measurement unit (IMU).

In some embodiments, the IMU can include one or more of a gyroscope, accelerometer, and magnetometer. The IMU can be affixed or integrated with the neuromodulation (e.g., neurostimulation) device 100. In an embodiment, the IMU is an off the shelf component. In addition to its ordinary meaning, the IMU can also include specific components as discussed below. For example, the IMU can include one more sensors capable of collecting motion data. In an embodiment, the IMU includes an accelerometer. In some embodiments, the IMU can include multiple accelerometers to determine motion in multiple axes. Furthermore, the IMU can also include one or more gyroscopes and/or magnetometer in additional embodiments. Since the IMU can be integrated with the neurostimulation device 100, the IMU can generate data from its sensors responsive to motion, movement, or vibration felt by the device 100. Furthermore, when the device 100 with the integrated IMU is worn by a user, the IMU can enable detection of voluntary and/or involuntary motion of the user.

The device 100 can optionally include user interface components, such as a feedback generator 114 and a display 116. The display 116 can provide instructions or information to users relating to calibration or therapy. The display 116 can also provide alerts, such an indication of response to therapy, for example. Alerts may also be provided using the feedback generator 114, which can provide haptic feedback to the user, such as upon initiation or termination of stimulation, for reminder alerts, to alert the user of a troubleshooting condition, to perform a tremor inducing activity to measure tremor motion, among others. Accordingly, the user interface components, such as the feedback generator 114 and the display 116 can provide audio, visual, and haptic feedback to the user.

Furthermore, the device 100 can include communications hardware 118 for wireless or wired communication between the device 100 and an external system, such as the user interface device discussed below. The communications hardware 118 can include an antenna. The communications hardware 118 can also include an Ethernet or data bus interface for wired communications.

While the illustrated figure shows several components of the device 100, some of these components are optional and not required in all embodiments of the device 100. In some embodiments, a system can include a diagnostic device or component that does not include neuromodulation functionality. The diagnostic device could be a companion wearable device connected wirelessly through a connected cloud server, and include, for example, sensors such as cardiac activity, skin conductance, and/or motion sensors as described elsewhere herein.

In some embodiments, the device 100 can also be configured to deliver one, two or more of the following: magnetic, vibrational, mechanical, thermal, ultrasonic, or other forms of stimulation instead of, or in addition to electrical stimulation. Such stimulation can be delivered via one, two, or more effectors in contact with, or proximate the skin surface of the patient. However, in some embodiments, the device is configured to only deliver electrical stimulation, and is not configured to deliver one or more of magnetic, vibrational, mechanical, thermal, ultrasonic, or other forms of stimulation.

Although several neurostimulation devices are described herein, in some embodiments nerves are modulated non-invasively to achieve neuro-inhibition. Neuro-inhibition can occur in a variety of ways, including but not limited to hyperpolarizing the neurons to inhibit action potentials and/or depleting neuron ion stores to inhibit firing action potentials. This can occur in some embodiments via, for example, anodal or cathodal stimulation, low frequency stimulation (e.g., less than about 5 Hz in some cases), or continuous or intermediate burst stimulation (e.g., theta burst stimulation). In some embodiments, the wearable devices have at least one implantable portion, which may be temporary or more long term. In many embodiments, the devices are entirely wearable and non-implantable.

## User Interface Device

Figure 1B illustrates communications between the neurostimulation device 100 and a user interface device 150 over a communication link 130. The communication link 130 can be wired or wireless. The neuromodulation (e.g., neurostimulation) device 100 is capable of communicating and receiving instructions from a user interface device 150. The user interface device 150 can include a computing device. In some embodiments, the user interface device 150 is a mobile computing device, such as a mobile phone, a smartwatch, a tablet, or a wearable computer. The user interface device 150 can also include server computing systems that are remote from the neurostimulation device. The user interface device 150 can include hardware processor(s) 152, a memory 154, display 156, and power electronics 158. In some embodiments, a user interface device 150 can also include one or more sensors, such as sensors described elsewhere herein. Furthermore, in some instances, the user interface device 150 can generate an alert responsive to device issues or a response to therapy. The alert may be received from the neurostimulation device 100.

In additional embodiments, data acquired from the one or more sensors 102 is processed by a combination of the hardware processor(s) 108 and hardware processor(s) 152. In further embodiments, data collected from one or more sensors 102 is transmitted to the user interface device 150 with little or no processing performed by the hardware processors 108. In some embodiments, the user interface device 150 can include a remote server that processes data and transmits signals back to the device 100 (e.g., via the cloud).

Figure 1C schematically illustrates a neuromodulation device and base station. The device can include a stimulator and detachable band including two or more working electrodes (positioned over the median and radial nerves) and a counter-electrode positioned on the dorsal side of the wrist. The electrodes could be, for example, dry electrodes or hydrogel electrodes. The base station can be configured to stream movement sensor and usage data on a periodic basis, e.g., daily and charge the device. The device stimulation bursting frequency can be calibrated to a lateral postural hold task "wing-beating" or forward postural hold task for a predetermined time, e.g., 20 seconds for each subject. Other non-limiting examples of device parameters can be as disclosed elsewhere herein.

In some embodiments, stimulation may alternate between each nerve such that the nerves are not stimulated simultaneously. In some embodiments, all nerves are stimulated simultaneously. In some embodiments, stimulation is delivered to the various nerves in one of many bursting patterns. The stimulation parameters may include on/off, time duration, intensity, pulse rate, pulse width, waveform shape, and the ramp of pulse on and off. In one preferred embodiment the pulse rate may be from about 1 to about 5000 Hz, about 1Hz to about 500Hz, about 5 Hz to about 50Hz, about 50 Hz to about 300 Hz, or about 150 Hz. In some embodiments, the pulse rate may be from 1 kHz to 20 kHz. A preferred pulse width may range from, in some cases, 50 to 500 $\mu$s (micro-seconds), such as approximately 300 $\mu$s. The intensity of the electrical stimulation may vary from 0 mA to 500 mA, and a current may be approximately 1 to 11 mA in some cases. The electrical stimulation can be adjusted in different patients and with different methods of electrical stimulation. The increment of intensity adjustment may be, for example, 0.1 mA to 1.0 mA. In one preferred embodiment the stimulation may last for approximately 10 minutes to 1 hour, such as approximately 10, 20, 30, 40, 50, or 60 minutes, or ranges including any two of the foregoing values. In some embodiments, a plurality of electrical stimuli can be delivered offset in time from each other by a predetermined fraction of multiple of a period of a measured rhythmic biological signal such as hand tremor, such as about ¼, ½, or ¾ of the period of the measured signal for example. Further possible stimulation parameters are described, for example, in U.S. Pat. 9,452,287 to Rosenbluth et al., U.S. Pat. No. 9,802,041 to Wong et al., PCT Pub. No. WO 2016/201366 to Wong et al., PCT Pub. No. WO 2017/132067 to Wong et al., PCT Pub. No. WO 2017/023864 to Hamner et al., PCT Pub. No. WO 2017/053847 to Hamner et al., PCT Pub. No. WO 2018/009680 to Wong et al., and PCT Pub. No. WO 2018/039458 to Rosenbluth et al., each of the foregoing of which are hereby incorporated by reference in their entireties.

Controller

Figure 2 illustrates a block diagram of an embodiment of a controller 200 that can be implemented with the hardware components described above with respect to Figures 1A-1C. The controller 200 can include multiple engines for performing the processes and functions described herein. The engines can include programmed instructions for performing processes as discussed herein for detection of input conditions and control of output conditions. The engines can be executed by the one or more hardware processors of the neuromodulation (e.g., neurostimulation) device100 alone or in combination with the patient monitor 150. The programming instructions can be stored in a memory 110. The programming instructions can be implemented in C, C++, JAVA, or any other suitable programming languages. In some embodiments, some or all of the portions of the controller 200 including the engines can be implemented in application specific circuitry such as ASICs and FPGAs. Some aspects of the functionality of the controller 200 can be executed remotely on a server (not shown) over a network. While shown as separate engines, the functionality of the engines as discussed below is not necessarily required to be separated. Accordingly, the controller 200 can be implemented with the hardware components described above with respect to Figures 1A-1C.

The controller 200 can include a signal collection engine 202. The signal collection engine 202 can enable acquisition of raw data from sensors embedded in the device, including but not limited to accelerometer or gyroscope data from the IMU 102. In some embodiments, the signal collection engine 202 can also perform signal preprocessing on the raw data. Signal preprocessing can include noise filtering, smoothing, averaging, and other signal preprocessing techniques to clean the raw data. In some embodiments, portions of the signals can be discarded by the signal collection engine 202.

The controller 200 can also include a feature extraction engine 204. The feature extraction engine 204 can extract relevant features from the signals collected by the signal collection engine 202. The features can be in time domain and/or frequency domain. For example, some of the features can include amplitude, bandwidth, area under the curve (e.g., power), energy in frequency bins, peak frequency, ratio between frequency bands, and the like. The features can be extracted using signal processing techniques such as Fourier transform, band pass filtering, low pass filtering, high pass filtering and the like.

The controller can further include a rule generation engine 206. The rule generation engine 206 can use the extracted features from the collected signals and determine rules that correspond to neurostimulation therapy. The rule generation engine 206 can automatically determine a correlation between specific extracted features and neurostimulation therapy outcomes. Outcomes can include, for example, identifying patients who will respond to the therapy (e.g., during an initial trial fitting or calibration process) based on tremor features of kinematic data (e.g., approximate entropy), predicting stimulation settings for a given patient (based on their tremor features) that will result in the best therapeutic effect (e.g., dose, where parameters of the dose or dosing of treatment include but are not limited to duration of stimulation, frequency and/or amplitude of the stimulation waveform, and time of day stimulation is applied), predicting patient tremor severity at a given point, predicting patient response over time, examining patient medication responsiveness combined with tremor severity over time, predicting response to transcutaneous or percutaneous stimulation, or implantable deep brain stimulation or thalamotomy based off of tremor features and severity over time, and predicting ideal time for a patient to receive transcutaneous or percutaneous stimulation, or deep brain stimulation or thalamotomy based off of tremor features and severity over time, predicting patient reported therapy outcomes or patient reported satisfaction using tremor features assessed kinematic measurements from the device; predicting patient response to undesirable user experience using tremor features assessed from kinematic measurements and patient usage logs from the device where undesirable user experiences can include but are not limited to device malfunctions and adverse events such as skin irritation or burn; predict patient response trends based on tremor severity where trends can be assessed across total number of sessions, within an individual patient, or across a population of patients; predicting or classifying subtypes of tremor to predicting patient response based on kinematic analysis of tremor features; predicting or classifying subtypes of tremor to provide guidance for individually optimized therapy parameters; predicting or classifying subtypes of tremor to optimize the future study design based on subtypes (e.g., selecting specific subtypes of essential tremor for a clinical study with specific design addressing therapy need for the subtype); and predict patient or customer satisfaction (e.g., net promoter score) based on patient response or other kinematic features from measure tremor motion. In some embodiments, essential tremor pathology can include, for example, a primarily cerebellar variant with Bergmann gliosis and Purkinje cell torpedoes, and a Lewy body variant, and a dystonic variant, and a multiple sclerosis variant, and a Parkinson disease variant.

In some embodiments, the neuromodulation, e.g., neurostimulation device can apply transcutaneous stimulation to a patient with tremor that is a candidate for implantable deep brain stimulation or thalamotomy. Tremor features and other sensor measurements of tremor severity will be used to assess response over a prespecified usage period, which could be 1 month or 3 months, or 5, 7, 14, 30, 60, or 90 days or more or less. Response to transcutaneous stimulation as assessed, for example, by algorithms described herein using sensor measurements from the device can advantageously provide input to a predictive model that provides an assessment of the patient's likelihood to respond to implantable deep brain stimulation or other implantable or non-implantable therapies.

In some embodiments, the neuromodulation, e.g., neurostimulation device or a secondary device with sensors can collect motion data, or data from other sensors, when a tremor inducing task is being performed. The patient can be directly instructed to perform the task, for example via the display on the device or audio. In some embodiments, features of tremor inducing tasks are stored on the device and used to automatically activate sensors to measure and store data to memory during relevant tremor tasks. The period of time for measuring and storing data can be, for example, 10, 20, 30, 60, 90, 120 seconds, or 1, 2, 3, 5, 10, 15, 20, 30 minutes, or 1, 2, 3, 4, 5, 6, 7, 8 hours or more or less, or ranges incorporating any two of the foregoing values. Based on a training set of data from a cohort of previous wearers with tremor or another condition, the feature extraction engine can detect features that are correlated with response to stimulation such that the patient or physician can be presented with a quantitative and/or qualitative likelihood of the patient responding or not responding to treatment. This data can be measured in some cases prior to prescribing the neuromodulation, e.g., neurostimulation or during a trial period. In another embodiment, features can be correlated with the type of tremor measured, such as resting tremor (associated with Parkinson's Disease), postural tremor, action tremor, intention tremor, rhythmic tremor (e.g., a single dominant frequency) or mixed tremor (e.g., multiple frequencies). The type of tremor most likely detected can be presented to the patient or physician as a diagnosis or informative assessment prior to receiving stimulation or to assess appropriateness of prescribing a neuromodulation, e.g., stimulation treatment. In another embodiment, various stimulation modes may be applied based on the tremor type determined; different modes could include changes in stimulation parameters, such as frequency, pulse width, amplitude, burst frequency, duration of stimulation, or time of day stimulation is applied. In one embodiment for a smartphone, tablet, or other device, the task to induce tremor can be included in an app that asks the patient to take a self-photograph, which has the patient perform a task that has both posture and intention actions.

In some embodiments, the neuromodulation, e.g., neurostimulation device or a secondary device with sensors can collect motion data, or data from other sensors, can measure data over a longer period of time, for example 1, 2, 3, 4, 5, 10, 20, 30 weeks, 1, 2, 3, 6, 9, 12 months, or 1, 2, 3, 5, 10 years or more or less, or ranges incorporating any two of the foregoing values, to determine features, or biomarkers, associated with the onset of tremor diseases, such as essential tremor, Parkinson's disease, dystonia, multiple sclerosis, etc. Biomarkers could include specific changes in one or more features of the data over time, or one or more features crossing a predetermined threshold. In some embodiments, features of tremor inducing tasks have been stored on the device and used to automatically activate sensors when those tremor inducing tasks are being performed, to measure and store data to memory during relevant times.

In some embodiments, the rule generation engine 206 relies on calibration instructions to determine rules between features and outcomes. The rule generation engine 206 can employ machine learning modeling along with signal processing techniques to determine rules, where machine learning modeling and signal processing techniques include but are not limited to: supervised and unsupervised algorithms for regression and classification. Specific classes of algorithms include, for example, Artificial Neural Networks (Perceptron, Back-Propagation, Convolutional Neural Networks, Recurrent Neural networks, Long Short-Term Memory Networks, Deep Belief Networks), Bayesian (Naive Bayes, Multinomial Bayes and Bayesian Networks), clustering (k-means, Expectation Maximization and Hierarchical Clustering), ensemble methods (Classification and Regression Tree variants and Boosting), instance-based (k-Nearest Neighbor, Self-Organizing Maps and Support Vector Machines), regularization (Elastic Net, Ridge Regression and Least Absolute Shrinkage Selection Operator), and dimensionality reduction (Principal Component Analysis variants, Multidimensional Scaling, Discriminant Analysis variants and Factor Analysis). In some embodiments, the controller 200 can use the rules to automatically determine outcomes. The controller 200 can also use the rules to control or change settings of the neurostimulation device, including but not limited to stimulation parameters (e.g., stimulation amplitude, frequency, patterned (e.g., burst stimulation), intervals, time of day, individual session or cumulative on time, and the like).

Accordingly, the rules can improve operation of the neuromodulation, e.g., neurostimulation device, and advantageously and accurately identify potential candidates for therapy and well as various disease state and therapy parameters over time. The generated rules can be saved in the memory 110 and/or memory 154. For example, the rules can be generated after calibration and stored prior to operation of the neurostimulation device 100. Accordingly, in some embodiments, a rule application engine 208 can apply the saved rules on new data collected by the IMU to determine outcomes or control the neuromodulation, e.g., neurostimulation device 100.

Specific examples of calibration and determination of rules is described in more detail below.

<u>Calibration</u>

In some embodiments, a neuromodulation device can include the ability to track a user's motion data for the purpose of gauging one, two, or more tremor frequencies of a patient. The patient could have a single tremor frequency, or in some cases multiple discrete tremor frequencies that manifest when performing different tasks. Once the tremor frequencies are observed, they can be used as one of many seminal input parameters to a customized neuromodulation therapy. The therapy can be delivered, e.g., transcutaneously, via one, two, or more nerves (e.g., the median and radial nerves, and/or other nerves disclosed elsewhere herein) in order to reduce or improve a condition of the patient, including but not limited to their tremor burden. In some embodiments, the therapy modulates afferent nerves, but not efferent nerves. In some embodiments, the therapy preferentially modulates afferent nerves. In some embodiments, the therapy does not involve functional electrical stimulation. The tremor frequency can be used to calibrate the patient's neuromodulation therapy, being used as a calibration frequency in some embodiments to set one or more parameters of the neuromodulation therapy, e.g., a burst envelope period. In some embodiments, the calibration frequency can be between, for example, about 4 Hz and about 12 Hz, between about 3 Hz and about 6 Hz, or about 3 Hz, 4 Hz, 5 Hz, 6 Hz, 7 Hz, 8 Hz, 9 Hz, 10 Hz, 11 Hz, or 12 Hz, or ranges including any two of the foregoing values.

In some embodiments, a hardware processor can be configured to perform any number of the following: obtain raw motion data by turning on the IMU transducer, e.g.,, the accelerometer; collect patient data over a selected time period (e.g., 10 seconds) (x/y/z axes); Perform a fast Fourier transform (FFT) on x-axis (yielding $\mathcal{F}$(x)); perform FFT on y-axis (yielding $\mathcal{F}$(y)); and/or perform FFT on z-axis (yielding $\mathcal{F}$(z)); derive the Total Frequency Domain (TFD) = $\Sigma$ { $\mathcal{F}$(x), $\mathcal{F}$(y), $\mathcal{F}$(z) }; and determine the calibration frequency as the highest value of TFD (e.g., between about 4 Hz and about 12 Hz in some cases).

Figure 3 illustrates an embodiment of a process 300 for calibration of neuromodulation, e.g., neurostimulation device 100. The process 300 can be implemented by any of the systems discussed above. The process 300 can be implemented to calibrate a specific neuromodulation, e.g., neurostimulation device 100 for a particular user or multiple neurostimulation devices across multiple users.

In an embodiment, the calibration process 300 begins at block 302 when the neuromodulation, e.g., neurostimulation device 100 is activated. The device can be activated in response to a user input. User input can be received via a push button or any other user interface such as the display 106 of the neuromodulation, e.g., neurostimulation device. In some embodiments, the neuromodulation, e.g., neurostimulation device 100 can be activated based on a signal received from a patient monitor 150 or another computing system.

Following activation of calibration, the controller 200 can begin collecting sensor data from the IMU 102. In an embodiment, the controller 200 can continue collecting sensor data for a period of 10 seconds, or about, at least about, or no more than about 1, 2, 3, 4, 5, 6, 7, 8, 9, 10, 11, 12, 13, 14, 15, 20, 25, 30, 40, 50, 60 seconds, ranges including any two of the aforementioned values, or other time periods. In some embodiments, the controller can continue collecting data for longer periods of time, such as a period of 1, 2, 3, 4, 5, 6, 7, 8 hours, or 12 or 24 hours, 1, 2, 3, 4 weeks, 1, 2, 3, 4, 5, 6 months or more, or indefinitely. After a period of data collection, the controller 200 can activate the electrodes 104 to apply neurostimulation at block 306. The controller 200 can acquire additional data from IMU 102 after the application of neurostimulation for a time period at block 308. In an embodiment, the post stimulation data collection time period is 10 seconds.

At block 310, the controller 200 can process the collected sensor data before and after stimulation to determine one or more outcomes discussed above, including determination of one or more rules.

In some instances, the calibration session can be processing and/or data intensive. Particularly, when the calibration is performed on the device, the onboard hardware processors and memory may not have enough processing or memory capabilities to process a large continuous data set. For example, assuming a sampling frequency of 104 Hz, a 10 second calibration can result in 1040 samples for each axis. Furthermore, each sample may require 4 bytes of data storage to store a floating point data. Therefore, the total data storage may correspond to 1040*3*4 = 12.48 KB. To reduce the memory usage, it may be advantageous in some instances to analyze the calibration signal in windows. The embedded processing devices are more efficient at analyzing samples that are power of 2. Accordingly, in some instances, 256 samples are selected, which corresponds to 2.46 seconds at a sampling rate of 104 samples/second. Other sampling rates may also be used. The corresponding data usage is 256*3*4 = 3.072 KB, which is significantly less than 12.48 KB. Note that the Nyquist frequency is 52 Hz, resulting in frequency bin resolution 0.40625 Hz. The windowed calibration can advantageously reduce the memory bandwidth needed within a neuromodulation device, potentially allowing for increased speed and real-time or near real-time processing.

Figure 3A illustrates a flow chart of a non-limiting embodiment of a process for performing windowed calibration. In some embodiments, the process can include any of the following: collecting a number of windows of x,y,z, accelerometry data, such as (5) 2.4 second windows; perform FFT on each window; sum the A+B+C of each window; average all (e.g., 5) FFTs; and perform peak detection. Accordingly, the processing is performed on windows and then combined as shown in Figure 3A, thereby reducing memory usage. In some instances, certain portions of the signal or windows affected by artifacts or noise may also be discarded (as described herein) to reduce processing requirements. Sampling a shorter time period, such as a window, over a long period of time may provide tremor frequency variability over a single capture. In some instances, a tremor frequency variability is an indicator for determining therapeutic effect of stimulation where a lower variability indicates a higher likelihood of the successful therapeutic outcome. The tremor frequency variability can be determined as change in frequencies where tremor peaks occur, change in power surrounding tremor peak frequencies (as described herein), or a combination of these metrics and any other that correlate with a property of the tremor peak. The calibration may also vary based on the tasks resulting in different calibration frequency for a particular task. In some instances, calibration may automatically determine the type of tasks performed by the user.

In some embodiments, a hardware processor can be configured to diversify calibration activity, and/or update session-by-session calibration. Peak tremor frequencies can be captured from kinematic measurement during different types of activities or tasks, such as 2, 3, 4, 5, 6, 7, 8, 9, 10, or more tasks. In some cases, a patient can have a first tremor frequency when performing a first activity or task, a second tremor frequency when performing a second activity or task, and/or a third tremor frequency when performing a third activity or task, etc. These tremor frequencies can in some cases differ up to 2.5 Hz or more, such as, for example, about 0.5 Hz, 1 Hz, 1.5 Hz, 2 Hz, 2.5 Hz, 3 Hz, or more or less, or ranges including any two of the foregoing values. Figure 3B is a graph that illustrates that in some cases, a patient's tremor frequency can vary across different tasks, including hold, action, a first drawing A task, a second drawing B task, and pouring, with respect to a postural hold task. Not to be limited by theory, treatment outcomes can be improved when neuromodulation, e.g., neurostimulation alternates between a first and second nerve, such as the median and radial nerve, at the frequency of tremor for a given task. As such, the patient response can improve by modifying the alternating frequency based on the task being performed before, during or after stimulation. This can involve, for example, moment-by-moment detection of different type of activity during stimulation; moment-by-moment extraction of tremor frequency during specific activities, either identified by the patient or identified by an algorithm based on unique kinematic features of a tremor-inducing task; and/or measuring tremor frequency during postural, action, and kinetic tremor tasks, storing the frequency of each task in memory on the device, and allowing the patient to select the type of task desired or modifying based on motion during neuromodulation, e.g., neurostimulation. For example, in some embodiments, the neuromodulation device can be configured to utilize a first calibration frequency for a first stimulation therapy during a first activity, and a second, different calibration frequency for a second stimulation therapy during a second, different activity.

## Data Collection

Figure 4 illustrates a flow chart of an embodiment of a process 400 for collecting data from IMU 102 and processing the collected data. The process 400 can be implemented by any of the systems described above.

In an embodiment, the process 400 begins at block 402 when the controller 200 receives sensor data from the IMU 102 in time domain. The sensor data can include raw accelerometer and/or gyroscope data, and/or other sensor data. In an embodiment, the sensor data can include data from each of the three axes. At block 404, the controller 200 can separate the received sensor data into four non-overlapping frames approximately 2.5 seconds in length, or other desired time periods. At block 406, the controller 406 can transform each frame into the frequency domain. At block 408, the controller 200 can combine the resulting spectra after frequency transform from each axis-frame. In an embodiment, the controller combines these spectra according to a user-specified input. Amplitude spectra can be calculated using the discrete-time Fourier transform. Further, power spectral density (PSD) estimates can be calculated using the periodogram with a Hann window. In an embodiment, the spectra from each of three axes for a given frame can combined using either the L1 or L2 norm across each frequency bin:

$$L1: P_{c,f} = \left|P_{x,f} + P_{y,f} + P_{z,f}\right|$$

$$L2: P_{c,f} = \sqrt{P_{x,f}^2 + P_{y,f}^2 + P_{z,f}^2}$$

where P(x,f) is the amplitude or power in frequency bin f in channel x.

Different and combinations of spectral features can be used to classify and predict tremor characteristics. For example, frequency components in the about 0 Hz to about 2.5 Hz can be used to detect periods of non-tremor movement, while frequency ranges between about 4 to about 12 Hz can be used to detect features related to the tremor, or about 3 to about 8 Hz for different variant of tremor, including but not limited to Parkinson disease. Kinematic features of the raw data signal from the IMU, such as regularity, amplitude and shape of the signal, can be used for classification.

At blocks 410 to 416, the controller 200 can examine the combined spectrum for each of the four frames for non-tremulous movement artifact. In an embodiment, the controller 200 calculates summed power in a low frequency band at block 410. The low frequency band can include frequencies between, for example, about 0 Hz and 2.5 Hz. These frequencies can be specified as input by the user. Further, at block 412, the controller 200 calculates energy in a second frequency band which is higher than the low frequency band. In an embodiment, the second frequency band includes frequencies between 4 Hz and 12 Hz, which include typical tremor frequencies, and typically 2.75 Hz surrounding the peak tremor frequency. In an embodiment, the controller 200 searches for a tremor spike in the second frequency band. The second frequency band can be user specified as an input.

At block 414, the controller 200 can compare the summed power in first and second frequency band. Comparisons can include dividing summed power in the second frequency band by the first frequency band. At block 416, the controller 200 can compare this ratio to a predetermined threshold to determine whether the particular frame can be used for further analysis. In an embodiment, if the controller 200 determines that the frame's summed power in the second frequency band is greater than the summed power in the first frequency band, or both the summed tremor band and the summed low-frequency band are less than a heuristically determined threshold, the controller 200 can determined that the frame is free from non-tremulous movement artifact. In some instance, the threshold can be entered by a user. Furthermore, in some instances, the threshold can be about 0.8. The threshold may be dependent on system or device parameters. In some instances, the threshold is about 0.5 to about 0.9. The frames that are identified as movement or artifact free can be used for further analysis. Frames with artifacts can be discarded or ignored by the controller 200. At block 416, the controller 200 can combine artifact free frames. In an embodiment, the controller 200 can average the remaining frames to yield a single spectrum.

At block 418, the controller 200 can use the processed data to determine metrics that can correlated to neurostimulation therapy outcomes. In one embodiment, the controller 200 can examine both acute (session timeframe) and chronic (entire treatment) effects. The controller can extract different metrics from the movement-free combined spectra from each pre- and post-stimulation recording. These metrics include amplitude/PSD at peak tremor frequency (typically constrained between 4-12 Hz, or between 3-8 Hz for Parkinson's tremor in some cases), summed amplitude/PSD in a band (typically 2.75 Hz wide) surrounding the peak tremor frequency, summed amplitude/PSD in a wide band (typically 4-12 Hz), or summed amplitude/PSD in a band surrounding the peak tremor frequency selected only from the pre-stimulation spectra. Parameters such as bandwidth and search frequencies can be predetermined or received from a user as an input. In some cases, Parkinsonian hand tremor is severe during resting (in contrast with essential tremor, which is severe during activity), such as when the hands are placed in one's lap, or when walking. Tremor measured during walking may involve different rules for extracting tremor signal and analysis. Tremor-inducing tasks for Parkinson's disease therapy can be different that of essential tremor. Non-limiting examples of tremor inducing tasks for Parkinson's disease can include, for example, resting hands in one's lap or on a table (e.g., when the hand, arms, or leg muscles are relaxed).

Figure 4A illustrates a flow chart of an embodiment of a process 401 for collecting data from IMU 102 and processing the collected data. The process 401 can be implemented by any of the systems described above. Process 401 can include any number of the blocks of process 400 of Figure 4, and also include additional blocks as part of or in addition to determining frames to use for analysis as in block 416. For example, process 401 can include at block 416A collecting sensor data in the time domain. The data can involve, for example, any number of approximate entropy, displacement, summary metrics for continuous data and/or functional data analysis (e.g., curve-fitting, functional PCA, filtering, mean, median, range, etc.), and/or event detection (e.g., movement classification) using both supervised and unsupervised methods. At block 416B, collected data can be separated into frames. At block 416C, metrics can be calculated based on all or a subset of frames of time series data (e.g., non-spectral analysis). In the case of approximate entropy for example, frames to be used for calculation can be determined based on intermediary outcome of PSD analysis flow (shown schematically as line 416').

When the above signal collection and pre-processing is used during calibration as discussed with respect to Figure 3, the controller 200 can examine certain exclusion criteria to determine if metrics from that session can be used in subsequent analyses. These analyses can be performed algorithmically, for example by comparing power associated with predefined frequency spectra known to be associated with voluntary and tremor motion. Features of the raw signal (i.e., in the time domain) or the frequency domain transformed version can be used generate new rules to classify whether frames of data should be included or excluded. These criteria can include at least one frame in pre- and post-stimulation recordings that was free from movement artifact, at least one frame in pre- and post-stimulation recordings that contained tremor, a minimum time between the end of stimulation and the post-stimulation recording, and a minimum time in between a pre-stimulation recording and the previous stimulation session.

Rule Generation

Figure 5A illustrates a flowchart of an embodiment of a process 500 for generation of rules to determine neurostimulation therapy outcomes. The process 500 can be implemented by any of the systems described above. As discussed above with respect to Figure 4, the controller 200 can collect and process data from IMU 402 (and/or other sensors). From this processed data, the controller 200 can extract features or metrics. In some embodiments, the controller 200 does not do motion artifact removal as discussed above with respect to blocks 410 - 416. Accordingly, the controller 200 can use all the frames for generation of rules.

The process 500 can begin at block 502 where the controller 200 can extract features from the collected signal. The features can include time domain features and/or frequency domain features. The features can be predetermined. For example, the controller 200 can search for certain features in the received signal or the processed signal. At block 504, the controller 200 can correlate the extracted features with a particular application or neurostimulation therapeutic outcomes. The correlation can include machine learning algorithms. Based on the correlations, the controller 506 can generate rules at block 506. In some embodiment, the controller 200 determines rules based on a training set data collected from many patient. In other embodiments, the controller 200 determines rules based on calibration data received from a particular patient. Furthermore, the controller 200 can update previously determined rules based on additional data processed while the neurostimulation device 100 is in use.

Following is an example of a rule generation process 500. In an embodiment, the controller 200 acquired accelerometer signals from the dominant hand of a user. The signals were divided into 2.5 second frames (50% overlap), and amplitude spectra were calculated for each axis-frame and combined using the L1-norm method described above with respect to Figure 4. In this particular example, no movement artifact detection was employed because experiments were not self-directed. From the combined spectrum from each frame, several different features were extracted as shown in Figure 5B. The controller 200 can combine these features across all frames from a given recording. Combination can include averaging the values from each frame and finding the minimum and maximum values across all frames. In an embodiment, all amplitude spectrum-based features were log transformed by the controller 200. Additionally, the duration of the recording and the type of task was also used by the controller 200 as features. For example, the controller 200 can receive as an input the type of task.

Using these features, the controller 200 created a random forest classifier to predict tremor severity, as measure by the clinical score, which assigns a value between 0 and 4 to each task, with 4 being most impaired. Out of approximately 1000 recordings, 100 were randomly chosen to have a clinical score distribution similar to the rest of the data and held out as a test set. With the rest of the data, 5-fold cross validation and random search was used to optimize the random forest hyperparameters. The optimized hyperparameters included the number of decision trees, maximum features for splitting a node, maximum tree depth, minimum data examples for splitting a node and minimum samples for creating a leaf. The search performed 50 iterations over random combinations of hyperparameters and selected the combination that yielded the best cross-validation accuracy. After selecting the best model hyperparameters, the model was retrained using all the data except the test set and was then evaluated using the held out data. Algorithms can be developed with data training sets from postural hold-based kinematics and controlled setting task-based kinematics, tested in real world time-locked task-based kinematics, and in ambulatory kinematics.

Rule Application

Rules can be stored in several ways, including but not limited to any number of the following: (1) After training on a cohort of data, rules could be stored in the cloud. Data would be transmitted periodically, e.g., every night, and the rules applied once data is transmitted. Changes to stimulation or results could be send back to the device or patient monitor after execution on the cloud; (2) Rules could be stored on the device or patient monitor in memory and executed on the processor. Data collected could be processed and rules applied in real time, after a measurement, or after stimulation is applied; and/or (3) Rule generation (and modification) could happen after each therapy session based on an assessment of tremor improvement and relevant features measured before, during and after each stimulation session.

Additional Applications and Working Examples

Non-invasive electrical stimulation of peripheral nerves, e.g., in the wrist, including the median and radial nerves, can result in decreased hand tremor in patients with essential tremor (TR). Motion sensors placed on the wrist can quantify tremor and provide kinematic measures that correlate with clinical ratings of tremor severity. Tremor severity can be quantified with wearable wrist sensor measurements during clinical ratings of hand tremor before and at distinct timepoints (e.g., up to 60 minutes or more) following a single session of non-invasive median and radial nerve stimulation.

Fifteen participants performed hand tremor-specific tasks from the Fahn-Tolosa-Marin Clinical Rating Scale (FTM-CRS), including finger to nose reach task, referred to as an action, as well as postural, drawing, and pouring tasks. Tremor severity was visually rated according to FTM-CRS during simultaneous kinematic measurement of wrist movement of the treated hand. Both improvement in FTM-CRS and kinematic measurements showed that non-invasive stimulation of the median and radial nerves at the wrist significantly improved symptomatic hand tremor out to 60 minutes following the end of stimulation. The measured kinematics were then found to correlate directly with visual clinical ratings, and were subsequently used to predict clinical ratings with a supervised machine learning regression model. The model can predict clinical ratings, on average, within ± 0.62 or better of visual clinical rating units, including but not limited to the postural hold task. The observed prediction error is smaller than resolution of the clinical rating scale (1 unit), which is as accurate or better than human raters. The combination of noninvasive neuromodulation and kinematic measurements of tremor immediately before and after a therapy session can advantageously treat and automatically classifying tremor reduction from stimulation in both the clinic and home settings.

The study procedure can include one or more single, in-office sessions. Three electrodes, e.g., hydrogel electrodes (or dry electrodes in other embodiments) were positioned transcutaneously to target the median and radial nerves of each participant, including a first (e.g., median) electrode, a second (e.g., radial) electrode, and a third (e.g., counter) electrode placed circumferentially on a device housing, such as a detachable band (Figure 6A). Active leads were placed over the median and radial nerves on the palmar and dorsal surface of the wrist and were connected to a stimulator (which can be integrated into or removably or otherwise attached to the neuromodulation device in some embodiments). A counter-electrode was connected to the dorsum of the wrist. Tremor frequency was measured using an IMU worn on the participant's wrist during a specified time, (e.g., ten second) forward postural hold with arms outstretched in front of the participant. The stimulation amplitude was increased by a desired amount, (e.g., 0.25 mA steps) until the participant reported paresthesia corresponding to the distributions of the median and radial nerves. Stimulation included a series of charge balanced biphasic pulses, 300 μs per phase, with a 50 μs period between the two phases, delivered at a frequency of 150 Hz. The stimulation alternated between the median and radial nerve at a frequency equal to each participant's tremor frequency (Figure 6B). The final stimulation amplitude was chosen to be the highest level of stimulation below muscle contraction that the participant found comfortable. A stimulation session consisted of 40 minutes of continuous stimulation at the chosen amplitude.

Kinematics of the wrist were measured while participants performed different tasks at a number of time points, e.g., 4 time points: Baseline (pre), 20 minutes into stimulation (During), immediately following stimulation (Post 0), 30 minutes after stimulation (Post 30) and 60 minutes after stimulation (Post 60) (Figure 6C). Multiple kinematic features for machine learning algorithm development were extracted from accelerometry to quantify tremor power and other tremor characteristics. To quantify tremor power, mean power was calculated in a frequency band corresponding to the strongest tremor oscillation in the accelerometer signal (e.g., peak power frequency ± 1.5Hz) in the e.g., 4-12Hz range. For each participant, the sensor expressed measured tremor power at each time point as a log ratio relative to the tremor power measured in the pre-stimulation recording (Pre), meaning that a reduction in tremor power results in a negative log ratio. This transformation was performed to compare the tremor reduction across all tasks irrespective of raw tremor magnitude in each task. The first and last second of each kinematic recording were discarded to exclude signal from preparatory movement. Spectral analysis of accelerometer data was performed using Welch's method (e.g., 2 second window, 50% overlap) to generate the power spectral density of the signal. The following features were extracted from the power spectral density plot (Figure 6D). Kinematic features in the time and frequency domain can be used as model input features. Peak tremor frequency ($Frequency_{peak}$) was defined as the frequency in the 4-12 Hz band with the highest power. Peak power

($Power_{peak}$) was defined as the power at the peak of the frequency with the highest power in the 4-12Hz band. Mean peak power ($Power_{peak\ mean}$) was defined as the mean power in a ± 1.5Hz window centered on the frequency with the highest power in the 4-12 Hz band. The summed peak power ($Power_{peak\ sum}$) was the sum of power in a power in a ± 1.5 Hz window centered on the frequency with the highest power in the 4-12 Hz band. Summed tremor band power ($Power_{4\text{-}12Hz\ sum}$) was the summed power in the 4-12 Hz frequency band. Spectral entropy ($Entropy_{spectral}$) is the entropy of the power spectral density in the 4-12 Hz range. Q factor was determined to be the peak frequency divided by the frequency range where the spectral power was above 50% of the peak power. Approximate entropy ($Entropy_{approx.}$) was calculated on the raw signal as a metric to quantify the amount of temporal regularity of time series data, using two continuous frames (5sec) with greatest tremor axis of accelerometer data for Pre and matched axis for Post. The length of compared runs was 2 and the threshold was 0.2 x standard deviations of the data.

Extracted features of the kinematic data were used to train a supervised machine learning model. The gradient boosted tree model was selected due to its robustness in regression and classification applications. Models were implemented using XGBoost in Python. The dataset was first randomly partitioned into a training set and a test set. To determine the impact of training set size, the proportion of data assigned to the training and test sets (20 - 80%) was systematically varied, and the prediction repeated a desired number of times, e.g., 250 times for each assignment group. Stratification was used to maintain the distribution of outcomes in both training and test sets. The training objective function was to minimize a square error with a L2 regularization term.

To identify the optimal values for model parameters, a grid search was performed over these parameters: number of trees (e.g., 5 to 55 in steps of 10), tree depth (e.g., 3 - 6 in steps of 1) and learning rate (e.g., 0.0001, 0.002, 0.004, and 0.0008). Default values in XGBoost were used for other model parameters. The accuracy of candidate models in the grid search parameter space was assessed using 3-fold cross-validation. The parameter set producing the highest training accuracy was used to construct the model to predict the clinical rating of the test data set.

The accuracy of the test prediction can be the mean absolute error (MAE) between the true and predicted clinical ratings. This was first calculated for sessions within each clinical rating group (e.g., 0 to 4), and then averaged across all clinical rating groups to account for differences in the number of data points in each group.

To determine the contribution of different kinematic features to the prediction, the feature importance of the models was examined. Feature gain was used as a measure of importance, which reflects improvement in classification from the feature of interest at each branch of the decision tree. The mean feature importance was calculated across the 250 models in each training/test group. Statistical analyses were performed using Python packages SciPy, NumPy, and visualized with Matplotlib. Non-parametric comparison (Wilcoxon signed rank test) and Pearson's correlation method were used.

Table 1 below illustrates examples of the change in tremor power for each task in log and equivalent % units (median and 95% confidence interval), Wicoxon signed rank test * p<0.05 and **p<0.01.

TABLE 1

| | During | | Post 0 min | | Post 30 min | | Post 60 min | |
|---|---|---|---|---|---|---|---|---|
| | $log_{10}$ | % | $log_{10}$ | % | $log_{10}$ | % | $log_{10}$ | % |
| Action | -0.22** ±0.12 | -40 -55 to -20 | -0.18** ±0.06 | -34 -43 to -25 | -0.20* ±0.10 | -37 -20 to -49 | -0.10* ±0.09 | -21 -37 to -2 |
| Drawing | -0.39** ±0.27 | -59 -78 to -23 | -0.33** ±0.24 | -53 -73 to -18 | -0.31** ±0.18 | -51 -68 to -26 | -0.35 ±0.32 | -55 -79 to -6 |
| Postural hold | -0.36* ±0.46 | -57 -85 to 24 | -0.60** ±0.57 | -75 -93 to -6 | -0.84** ±0.36 | -86 -94 to -67 | -0.40* ±0.25 | -60 -78 to -29 |
| Pouring | -0.15** ±0.10 | -28 -43 to -1 | -0.36** ±0.14 | -57 -68 to -41 | -0.26* ±0.13 | -45 -59 to -26 | -0.24* ±0.12 | -43 -56 to -25 |

A reduction in sensor measured tremor kinematics was observed during (28-59% improvement across tasks) immediately following stimulation (34 to 75% improvement) for up to 60 minutes post-stimulation (21 to 60% improvement). A persistent and significant negative log ratio was observed at time points during, immediately following, after 30 minutes, and at 60 minutes relative to pre-stimulation session (Figures 7A-7D, Table 1). The time course in the action task showed a significant decrease at all timepoints out to an hour (Figure 7A, Table 1). The drawing (e.g., spiral drawing) time course showed a significant decrease in tremor out to 30 minutes after stimulation offset (Figure 7B, Table 1). The time course in both the forward postural hold and pouring tasks showed a significant decrease in tremor out to 60 minutes post stimulation (Figure 7C, 7D, Table 1). No adverse events were reported.

A significant correlation was found between mean peak tremor power ($Power_{peak\,mean}$) and clinical visual ratings for the finger-to-nose action task (Fig. 8A; $R^2$=0.40, $p<10^{-4}$) spiral drawing (Fig. 8B; $R^2$=0.57, $p<10^{-4}$), forward postural hold (Fig. 8C; $R^2$=0.58, $p<10^{-4}$) and a somewhat lesser degree in pouring (Fig. 8D). Each point in Figs. 8A-8D indicates the value for a single session. Peak tremor power refers to the power of wrist acceleration between 4 and 12Hz (mean power for the window around peak frequency ± 1.5Hz) . The clinical ratings in the plots have been jittered to improve visibility of each point.

Figs. 9A-9D illustrate that sensor measured kinematics can predict clinical ratings across tasks. For each subpanel (Figs. 9A-9D), the left column shows normalized confusion matrices for the predictions from one model using 50% of data for training in each task. For sessions in each true clinical rating group, the values indicate the percentage of sessions with the predicted rating. Prediction accuracy as measured by mean absolute error (MAE), balanced for clinical rating group size: Fig. 9A. Action task = 0.62, Fig. 9B. Drawing task = 0.78, Fig. 9C. Postural hold task = 0.42 and Fig. 9D. Pouring task = 0.54. The right column shows the importance of model input features sorted by the each feature's information gain.

Predicted clinical ratings shown in Figs. 9A-9D reflected their true clinical rating as shown by the diagonal, and off-diagonal, having the highest values in the confusion matrix (Fig. 9 left column). The prediction accuracy of the models ranged from ± 0.42 - 0.81 clinical rating units across tasks. This indicates the model can unexpectedly and advantageously predict the clinical rating, on average within ± 0.62 of actual clinical rating units, with the prediction being most accurate for the postural hold task (± 0.42 clinical units). The output of the model may also be expressed as probability. In some instances, the output of the model can indicate the length of time that the therapy will be effective before another stimulation therapy is needed. Accordingly, the model improves therapy and treatment of users. The model can also improve the neurostimulation device by determining appropriate usage of the device.

The distribution of prediction accuracy was determined, and how it is affected by the proportion of training. The prediction accuracy of the models was not strongly affected by training data size. By repeatedly building models using varying proportions of the data for training and testing (MAE: 0.48 - 0.64 for 80% training data), it was found that the prediction accuracy increased with greater proportion of training data (R -0.29 to -0.1, $p<0.001$, and explained variance 0.01 to 0.08) for each task. However, the improvements were small, indicating the models were reliable and not overfitting, even with small training sets.

It was found that tremor reduction during and following median and radial nerve stimulation at the wrist lasted as long as measured, out to about or at least about 60 minutes following about or at least about 40 minutes of stimulation. Significant and persistent tremor reduction was evident across the different FTM-CRS tremor tasks, including action, drawing, postural hold, and pouring tasks. These results show that there is an unexpectedly superior durable effect following a single stimulation session.

Mobile sensing technology can be advantageous in monitoring tremor status. Sensor kinematic features that map to clinical visual ratings can reduce subjectivity and variance in evaluating tremor severity and provide insight into tremor status. It was unexpectedly and advantageously found that tremor severity measured with accelerometers on the wrist correlated with visual ratings. Angular velocities can also correlate with visual ratings in some cases. Certain tasks, such as postural hold, can be especially advantageous in some cases to capture information about tremor severity using movement sensors, although other tasks as disclosed, for example herein can also be utilized instead or in combination.

Systems and methods herein can predict clinical ratings, on average within ± 0.48 - 0.64 of clinical ratings which is a meaningful resolution for at-home measurements. The observed prediction error is smaller than the resolution of this clinical rating scale (1 unit), which is as accurate or better than human raters. The accuracy of prediction and the importance of kinematic features to the prediction varied by the size of training data, but the effects were weak (explained variance 0.01 to 0.08), indicating prediction accuracy was not sensitive to small training datasets. The features that went into these models to predict clinical ratings can also be utilized to predict therapeutic response over time and at home. The features are shown, for example, in Figure 9.

In some embodiments, neuromodulation therapy can be combined with real time wrist kinematic measurements to treat and predict tremor reduction in a clinic or other setting. As one example, median and radial nerve stimulation at the wrist has a durable effect on essential tremor following stimulation for at least about 1, 2, 3, 4, 5, 6, 7, 8, 9, 10, 11, 12, 15, 18, 24 hours, or more, or ranges including any two of the foregoing values. Noninvasive neuromodulation can be combined with sensor data recorded before, during, and immediately following stimulation to understand therapeutic response, such as in an at-home setting.

Fig. 10 is a graph illustrating a correlation between motion data (e.g., accelerometry) and clinical scale (Tremor Research Group Essential Tremor Rating Assessment Scale (TETRAS)). Accelerometry data of tremor motion was collected in three 12 second recordings while the patient performed a tremor-inducing task while TETRAS ratings were performed simultaneously by a trained physician. Tremor severity from accelerometry was calculated as tremor power and plotted on a logarithmic scale. To quantify tremor power, mean power was calculated in a frequency band corresponding to the strongest tremor oscillation in the accelerometer signal (peak power frequency +/- 1.5Hz) in the 4-12Hz range.

Fig. 11 demonstrates that prediction of patient response can improve with data collected over a longer period of time. In some embodiments, patient response can be predicted after one therapy session. However, in some embodiments, patient response is not predicted after one therapy session, and can require about or at least about 1 week, 2 weeks, 3 weeks, 1 month, 2 months, 3 months, or more of therapy sessions, and/or 2, 3, 4, 5, 6, 7, 8, 9, 10, 15, 20, 25, 30, 35, 40, 45, 50, or more therapy sessions of a specified length (e.g., 30 minutes, 40 minutes, 60 minutes, or more or less in some embodiments). Fig. 11 plots correlation of patient response averaged over 1 day / 1 week / 1 month and patient response randomly picked over 1 day / 1 week/ 1 month of outcome during a 3-month study, indicating that correlation to patient response improves over time from 1 day to 1 week to 1 month.

Fig. 12 further demonstrates how patient response on first day / first week / first month of therapy predicts response over all sessions based on average outcome during a 3-month study, and indicates that correlation to patient response improves over time from 1 day to 1 week to 1 month.

Fig. 13 illustrates that the efficacy of neuromodulation therapy is high for both patients on and off medications (e.g., tremor medications) at the time of neuromodulation therapy, and especially for patients off medications.

Fig. 14 is a graph illustrating that patient measured therapy outcome and sensor measured kinematic improvement were correlated, illustrating mean PSI (patient selfreporting of improvement) after each session was correlated with kinematic improvement, as measured via the median improvement ratio (log10(pre/post) == fold change. In some embodiments, correlation between kinematic improvement and other user satisfaction/rating metrics (e.g., NPS, QUEST) can also be made using systems and methods as disclosed herein.

Figs. 15A-15D illustrate examples of endpoints for sensor measures, according to some embodiments. Fig. 15A illustrates representative time series and consolidated frequency spectrum data for a single pre-stimulation (top) and post-stimulation (bottom) recording. Fig. 15B illustrates a visual rating correlation with simultaneous sensor recording during postural hold. Fig. 15C illustrates individual average percent improvement, and population level average improvement in tremor severity at-home for subjects over three months. Fig. 15D illustrates the average change in tremor severity per session for all subjects, displayed on a logarithmic scale.

## Terminology

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. The methods disclosed herein include certain actions taken by a practitioner; however, they can also include any third-party instruction of those actions, either expressly or by implication. For example, actions such as "percutaneously stimulating an afferent peripheral nerve" includes "instructing the stimulation of an afferent peripheral nerve."

## Claims

**1.** A neurostimulation device (100) for determining whether a user is a candidate for deep brain stimulation or thalamotomy, the neurostimulation device comprising:

one or more electrodes (102) configured to generate neurostimulation;
one or more sensors (112) configured to detect tremor features after application of the neurostimulation, wherein the neurostimulation comprises transcutaneous neurostimulation;
a controller (108) configured to provide data based on the one or more sensors,
wherein the data comprises an assessment of a response of the user to the neurostimulation over a prespecified usage period; **characterized in that** the assessment of the response indicates a likelihood that the user will respond to deep brain stimulation or thalamotomy.

**2.** The device of Claim 1, wherein the device is configured for placement on a leg or arm.

3. The device of Claim 1, wherein the device is configured for placement on a wrist.

4. The device of any one of the preceding claims, wherein at least one of the one or more electrodes is configured to generate transcutaneous neurostimulation of a median nerve.

5. The device of any one of the preceding claims, wherein at least one of the one or more electrodes is configured to generate transcutaneous neurostimulation of a radial nerve.

6. The device of any one of the preceding claims, wherein the one or more electrodes is configured to generate transcutaneous neurostimulation of at least a median nerve and a radial nerve.

7. The device of any one of the preceding claims, wherein the controller comprises a rule generation engine configured to determine at least one correlation between the response of the user to the neurostimulation and the likelihood that the user will respond to deep brain stimulation or thalamotomy.

8. The device of any one of the preceding claims, wherein the assessment of the response indicates a likelihood that the user will respond to deep brain stimulation.

9. The device of any one of the preceding claims, wherein the assessment of the response indicates a likelihood that the user will respond to thalamotomy.

10. The device of any one of the preceding claims, wherein the one or more tremor features are kinematic features.

11. The device of any one of the preceding claims, wherein the controller is configured to examine effects of the neurostimulation per a treatment session timeframe and/or through an entire treatment course.

12. The device of any one of the preceding claims, wherein the response of the user to the neurostimulation is assessed by an algorithm that provides input to a predictive model.

13. The device of any one of the preceding claims, wherein the assessment of a response of the user to the neurostimulation comprises measurement of tremor severity.

14. The device of any one of the preceding claims, wherein the one or more tremor features comprise kinematic measurements.

15. The device of any one of the preceding claims, wherein the electrodes are non implantable.

**Patentansprüche**

1. Neurostimulationsvorrichtung (100) zum Bestimmen, ob ein Benutzer ein Kandidat für eine tiefe Hirnstimulation oder eine Thalamotomie ist, wobei die Neurostimulationsvorrichtung umfasst:

   eine oder mehrere Elektroden (102), die zum Erzeugen einer Neurostimulation konfiguriert sind;
   einen oder mehrere Sensoren (112), die zum Erfassen von Tremormerkmalen nach dem Anwenden der Neurostimulation konfiguriert sind,
   wobei die Neurostimulation eine transkutane Neurostimulation umfasst;
   eine Steuervorrichtung (108), die so konfiguriert ist, dass sie Daten auf Grundlage der einen oder mehreren Sensoren bereitstellt,
   wobei die Daten eine Bewertung einer Reaktion des Benutzers auf die Neurostimulation über einen vorab festgelegten Nutzungszeitraum umfassen;
   **dadurch gekennzeichnet, dass**
   die Bewertung der Reaktion eine Wahrscheinlichkeit anzeigt, dass der Benutzer auf eine tiefe Hirnstimulation oder eine Thalamotomie anspricht.

2. Vorrichtung nach Anspruch 1, wobei die Vorrichtung für die Positionierung an einem Bein oder Arm konfiguriert ist.

3. Vorrichtung nach Anspruch 1, wobei die Vorrichtung für die Positionierung an einem Handgelenk konfiguriert ist.

4. Vorrichtung nach einem der vorstehenden Ansprüche, wobei mindestens eine der einen oder mehreren Elektroden so konfiguriert ist, dass sie eine transkutane Neurostimulation eines Nervus medianus erzeugt.

5. Vorrichtung nach einem der vorstehenden Ansprüche, wobei mindestens eine der einen oder mehreren Elektroden so konfiguriert ist, dass sie eine transkutane Neurostimulation eines Nervus radialis erzeugt.

6. Vorrichtung nach einem der vorstehenden Ansprüche, wobei die eine oder die mehreren Elektroden so konfiguriert sind, dass sie eine transkutane Neurostimulation mindestens eines Nervus medianus und eines Nervus radialis erzeugen.

7. Vorrichtung nach einem der vorstehenden Ansprüche, wobei die Steuervorrichtung eine Regelgenerierungsmaschine umfasst, die so konfiguriert ist, dass sie mindestens eine Korrelation zwischen der Reaktion des Benutzers auf die Neurostimulation und der Wahrscheinlichkeit, dass der Benutzer auf eine tiefe Hirnstimulation oder eine Thalamotomie anspricht, bestimmt.

8. Vorrichtung nach einem der vorstehenden Ansprüche, wobei die Bewertung der Reaktion eine Wahrscheinlichkeit anzeigt, dass der Benutzer auf eine tiefe Hirnstimulation anspricht.

9. Vorrichtung nach einem der vorstehenden Ansprüche, wobei die Bewertung der Reaktion eine Wahrscheinlichkeit anzeigt, dass der Benutzer auf eine Thalamotomie anspricht.

10. Vorrichtung nach einem der vorstehenden Ansprüche, wobei das eine oder die mehreren Tremormerkmale kinematische Merkmale sind.

11. Vorrichtung nach einem der vorstehenden Ansprüche, wobei die Steuervorrichtung so konfiguriert ist, dass sie die Auswirkungen der Neurostimulation pro Behandlungssitzung und/oder über einen gesamten Behandlungsverlauf hinweg untersucht.

12. Vorrichtung nach einem der vorstehenden Ansprüche, wobei die Reaktion des Benutzers auf die Neurostimulation durch einen Algorithmus bewertet wird, der Eingaben für ein Vorhersagemodell bereitstellt.

13. Vorrichtung nach einem der vorstehenden Ansprüche, wobei die Bewertung einer Reaktion des Benutzers auf die Neurostimulation die Messung der Tremor-Schwere umfasst.

14. Vorrichtung nach einem der vorstehenden Ansprüche, wobei das eine oder die mehreren Tremormerkmale kinematische Messungen umfassen.

15. Vorrichtung nach einem der vorstehenden Ansprüche, wobei die Elektroden nicht implantierbar sind.

## Revendications

1. Dispositif de neurostimulation (100) pour déterminer si un utilisateur est un candidat pour stimulation cérébrale profonde ou thalamotomie, le dispositif de neurostimulation comprenant :

   une ou plusieurs électrodes (102) configurées pour générer une neurostimulation ;
   un ou plusieurs capteurs (112) configurés pour détecter des caractéristique de tremblement après l'application de la neurostimulation,
   dans lequel la neurostimulation comprend une neurostimulation transcutanée ;
   une unité de commande (108) configurée pour fournir des données sur la base de l'un ou des plusieurs capteurs, dans lequel les données comprennent une évaluation d'une réponse de l'utilisateur à la neurostimulation au cours d'une période d'utilisation préalablement spécifiée ; **caractérisé en ce que**
   l'évaluation de la réponse indique une probabilité que l'utilisateur répondra à la stimulation cérébrale profonde ou thalamotomie.

2. Dispositif de la revendication 1, dans lequel le dispositif est configuré pour être positionné sur une jambe ou un bras.

3. Dispositif de la revendication 1, dans lequel le dispositif est configuré pour être positionné sur un poignet.

**4.** Dispositif de l'une quelconque des revendications précédentes, dans lequel au moins une de l'une ou des plusieurs électrodes est configurée pour générer une neurostimulation transcutanée d'un nerf médian.

**5.** Dispositif de l'une quelconque des revendications précédentes, dans lequel au moins une de l'une ou des plusieurs électrodes est configuré pour générer une neurostimulation transcutanée d'un nerf radial.

**6.** Dispositif de l'une quelconque des revendications précédentes, dans lequel l'une ou les plusieurs électrodes sont configurées pour générer une neurostimulation transcutanée d'au moins un nerf médian et un nerf radial.

**7.** Dispositif de l'une quelconque des revendications précédentes, dans lequel l'unité de commande comprend un moteur de génération de règle configuré pour déterminer au moins une corrélation entre la réponse de l'utilisateur à la neurostimulation et la probabilité que l'utilisateur répondra à la stimulation cérébrale profonde ou thalamotomie.

**8.** Dispositif de l'une quelconque des revendications précédentes, dans lequel l'évaluation de la réponse indique une probabilité que l'utilisateur répondra à la stimulation cérébrale profonde.

**9.** Dispositif de l'une quelconque des revendications précédentes, dans lequel l'évaluation de la réponse indique une probabilité que l'utilisateur répondra à la thalamotomie.

**10.** Dispositif de l'une quelconque des revendications précédentes, dans lequel l'une ou les plusieurs caractéristique de tremblement sont des caractéristiques cinématiques.

**11.** Dispositif de l'une quelconque des revendications précédentes, dans lequel l'unité de commande est configurée pour examiner des effets de la neurostimulation pour chaque période de session de traitement et/ou durant une série de traitements entière.

**12.** Dispositif de l'une quelconque des revendications précédentes, dans lequel la réponse de l'utilisateur à la neurostimulation est évaluée par un algorithme qui fournit une entrée à un modèle prédictif.

**13.** Dispositif de l'une quelconque des revendications précédentes, dans lequel l'évaluation d'une réponse de l'utilisateur à la neurostimulation comprend la mesure de sévérité de tremblement.

**14.** Dispositif de l'une quelconque des revendications précédentes, dans lequel l'une ou les plusieurs caractéristique de tremblement comprennent des mesures cinématiques.

**15.** Dispositif de l'une quelconque des revendications précédentes, dans lequel les électrodes ne sont pas implantables.

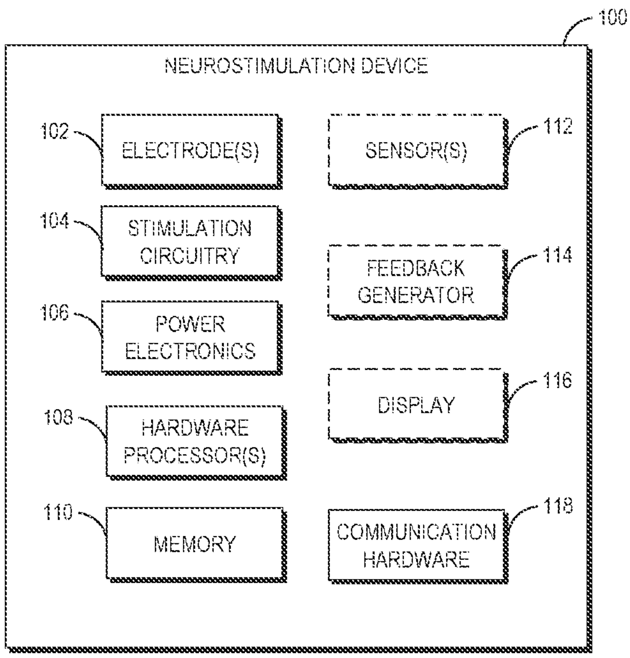
100
NEUROSTIMULATION DEVICE
102
ELECTRODE(S)
104
STIMULATION CIRCUITRY
106
POWER ELECTRONICS
108
HARDWARE PROCESSOR(S)
110
MEMORY
SENSOR(S)
112
FEEDBACK GENERATOR
114
DISPLAY
116
COMMUNICATION HARDWARE
118

FIG. 1A

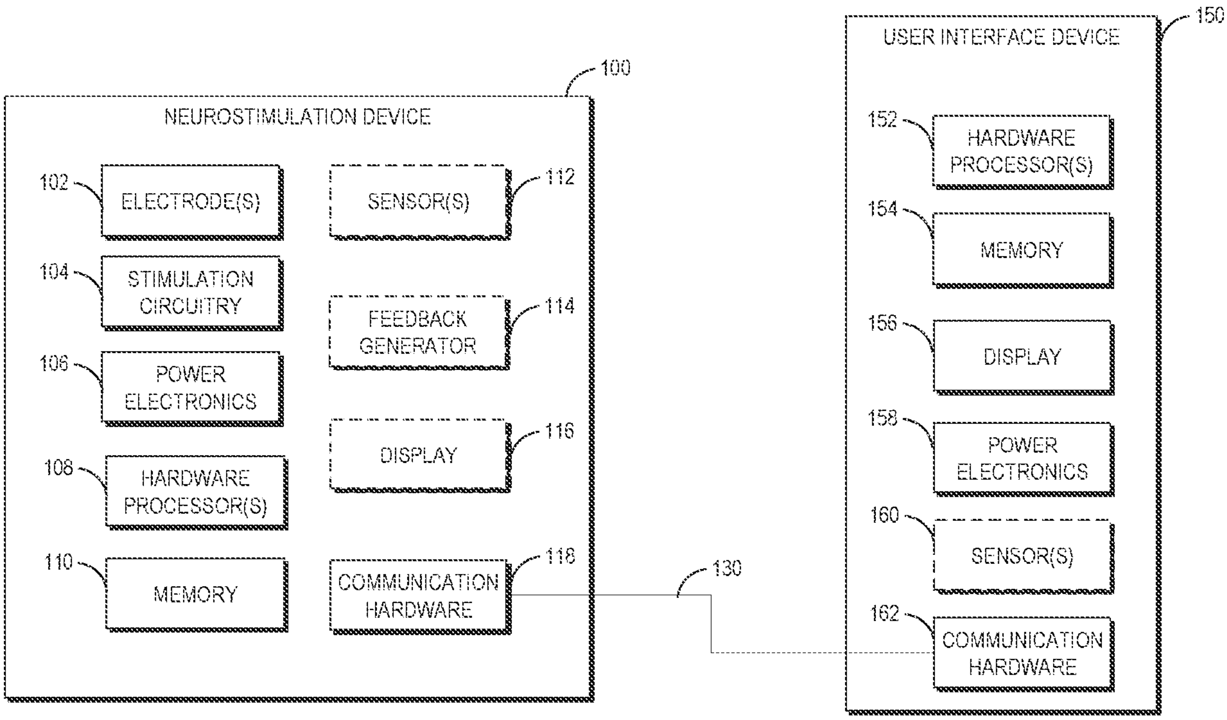
NEUROSTIMULATION DEVICE
100
102
ELECTRODE(S)
104
STIMULATION CIRCUITRY
106
POWER ELECTRONICS
108
HARDWARE PROCESSOR(S)
110
MEMORY
SENSOR(S)
112
FEEDBACK GENERATOR
114
DISPLAY
116
COMMUNICATION HARDWARE
118
130
USER INTERFACE DEVICE
150
152
HARDWARE PROCESSOR(S)
154
MEMORY
156
DISPLAY
158
POWER ELECTRONICS
160
SENSOR(S)
162
COMMUNICATION HARDWARE

FIG. 1B

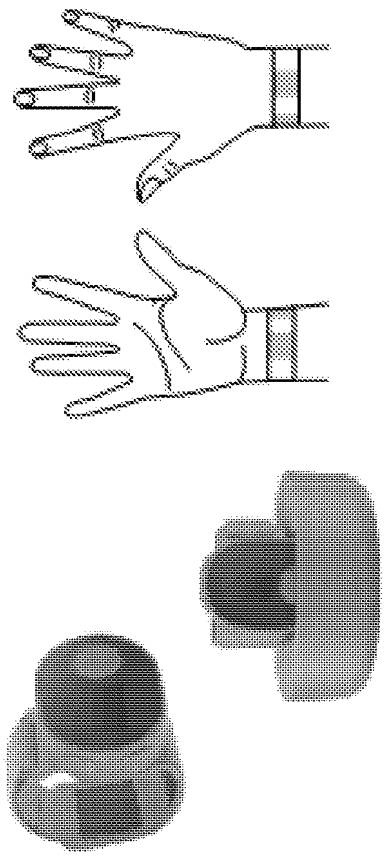

FIG. 1C

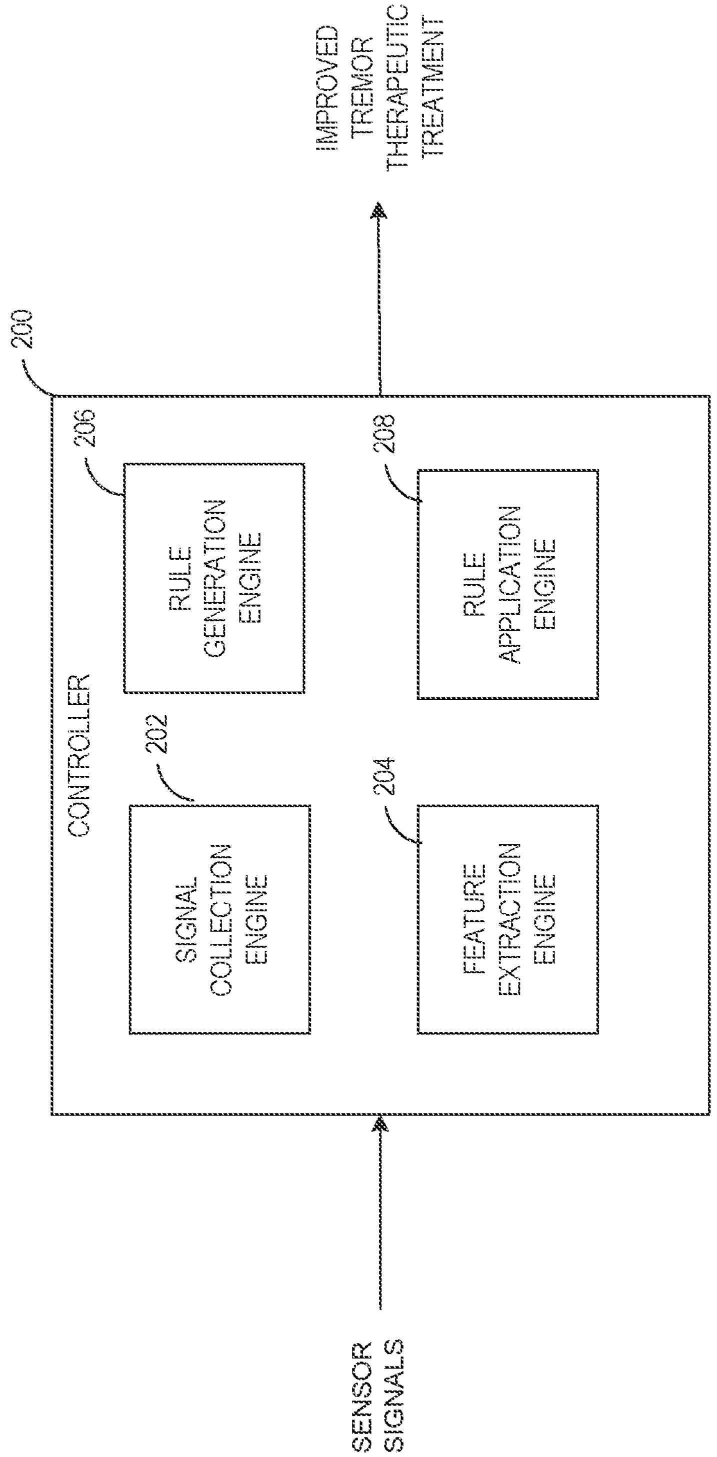
200
CONTROLLER
SIGNAL COLLECTION ENGINE
202
RULE GENERATION ENGINE
206
FEATURE EXTRACTION ENGINE
204
RULE APPLICATION ENGINE
208
SENSOR SIGNALS
IMPROVED TREMOR THERAPEUTIC TREATMENT

FIG. 2

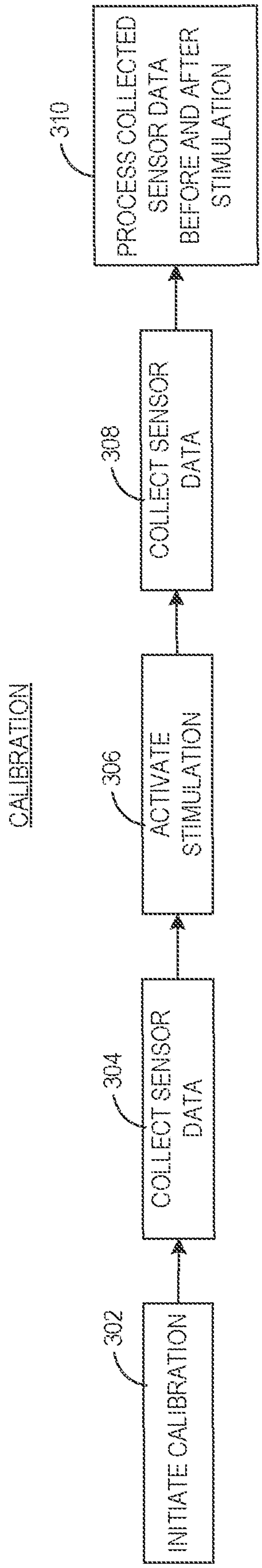
CALIBRATION
302
INITIATE CALIBRATION
304
COLLECT SENSOR DATA
306
ACTIVATE STIMULATION
308
COLLECT SENSOR DATA
310
PROCESS COLLECTED SENSOR DATA BEFORE AND AFTER STIMULATION

FIG. 3

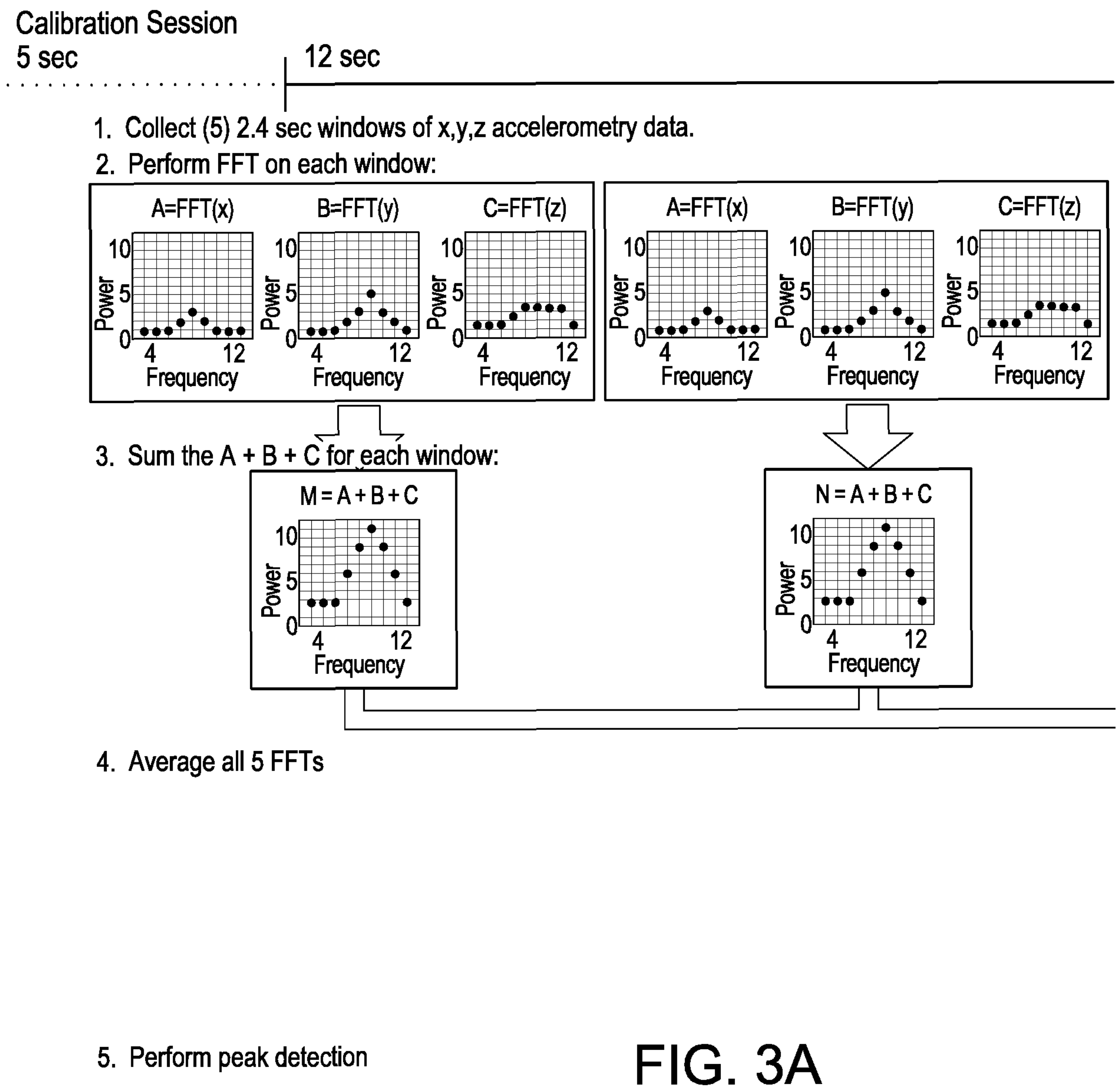

Calibration Session
5 sec
12 sec
1. Collect (5) 2.4 sec windows of x,y,z accelerometry data.
2. Perform FFT on each window:
A=FFT(x)
B=FFT(y)
C=FFT(z)
Power
Frequency
10
5
0
4
12
3. Sum the A + B + C for each window:
M = A + B + C
N = A + B + C
4. Average all 5 FFTs
5. Perform peak detection


FIG. 3A

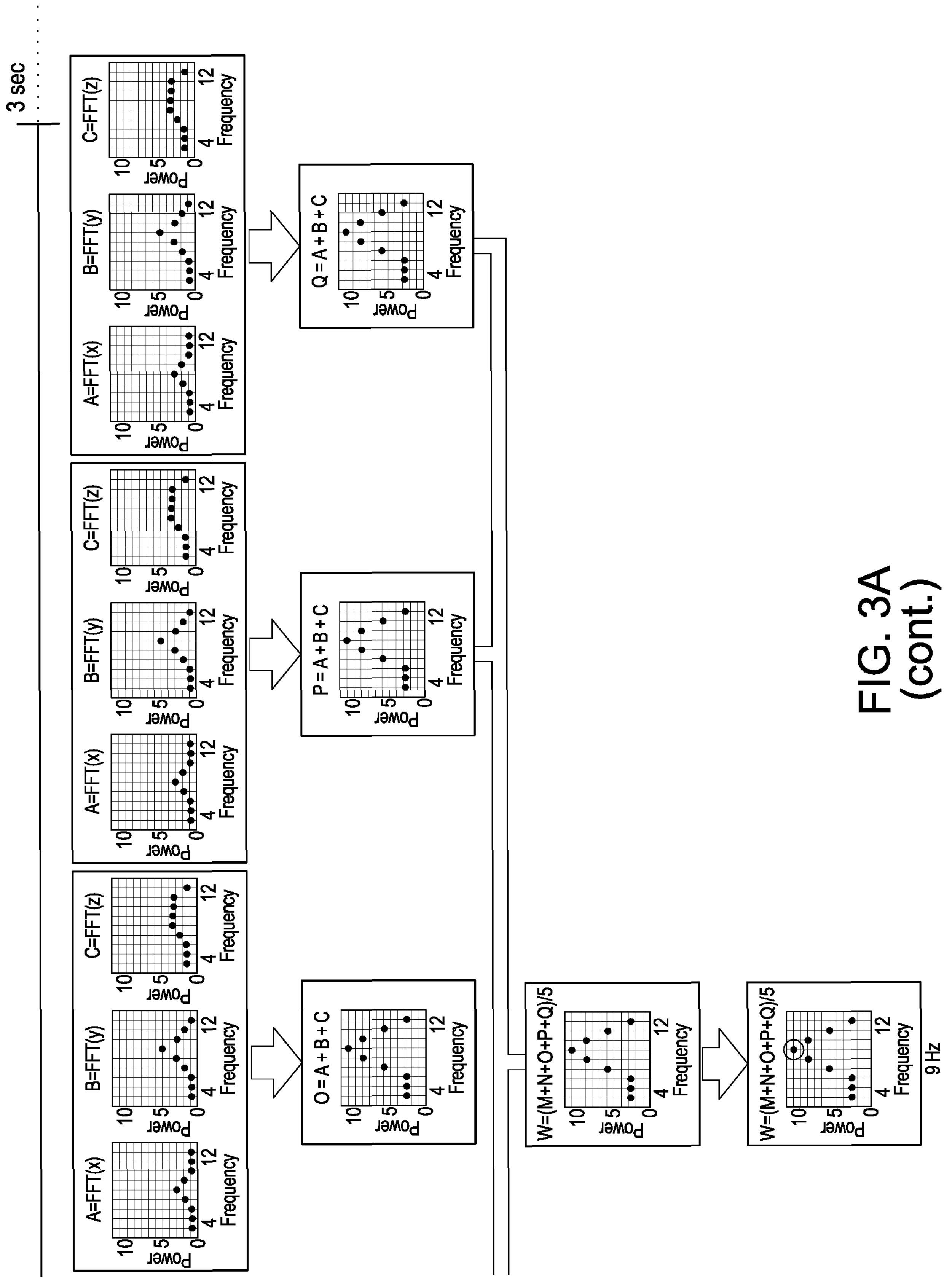
3 sec
A=FFT(x)
B=FFT(y)
C=FFT(z)
Power
Frequency
10
5
0
4
12
O = A + B + C
P = A + B + C
Q = A + B + C
W=(M+N+O+P+Q)/5
9 Hz

FIG. 3A (cont.)

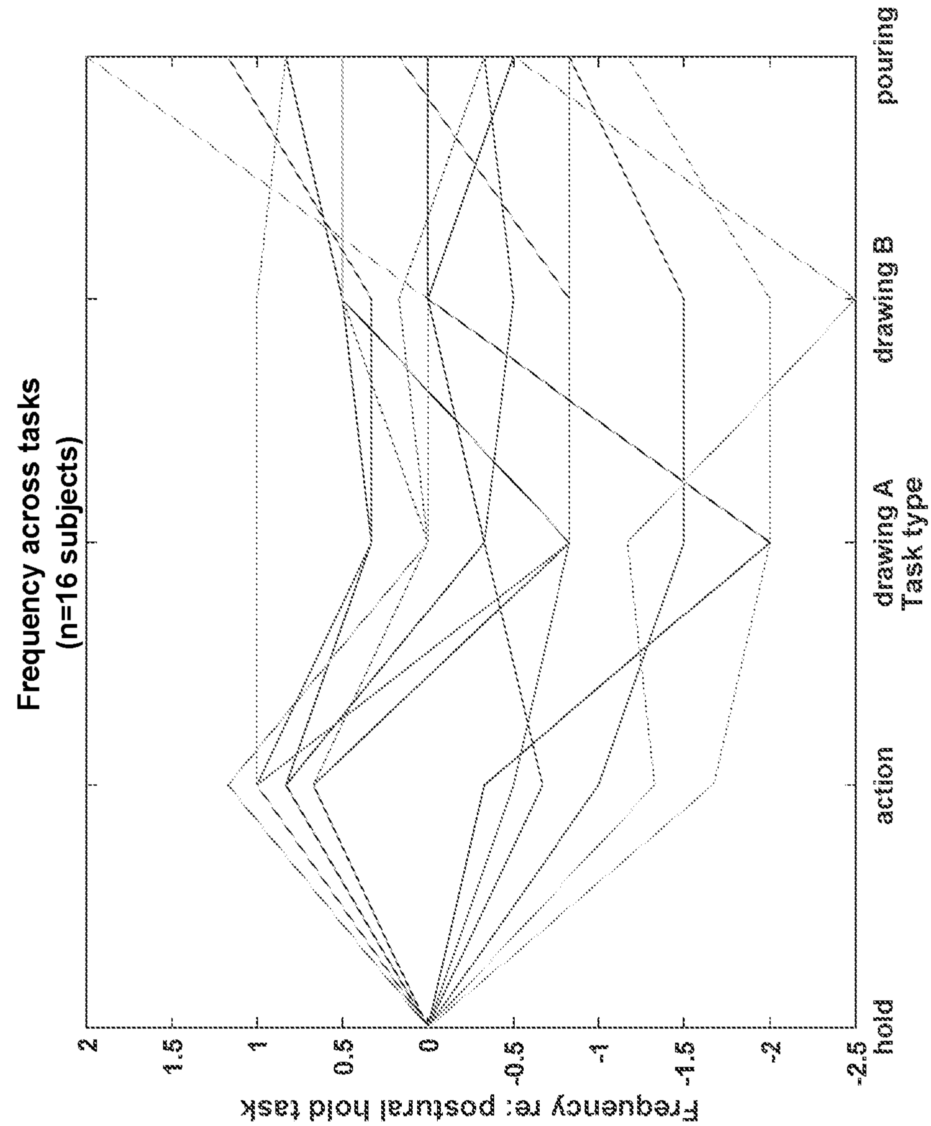

Frequency across tasks
(n=16 subjects)
Frequency re: postural hold task
2
1.5
1
0.5
0
-0.5
-1
-1.5
-2
-2.5
hold
action
drawing A
drawing B
pouring
Task type


FIG. 3B

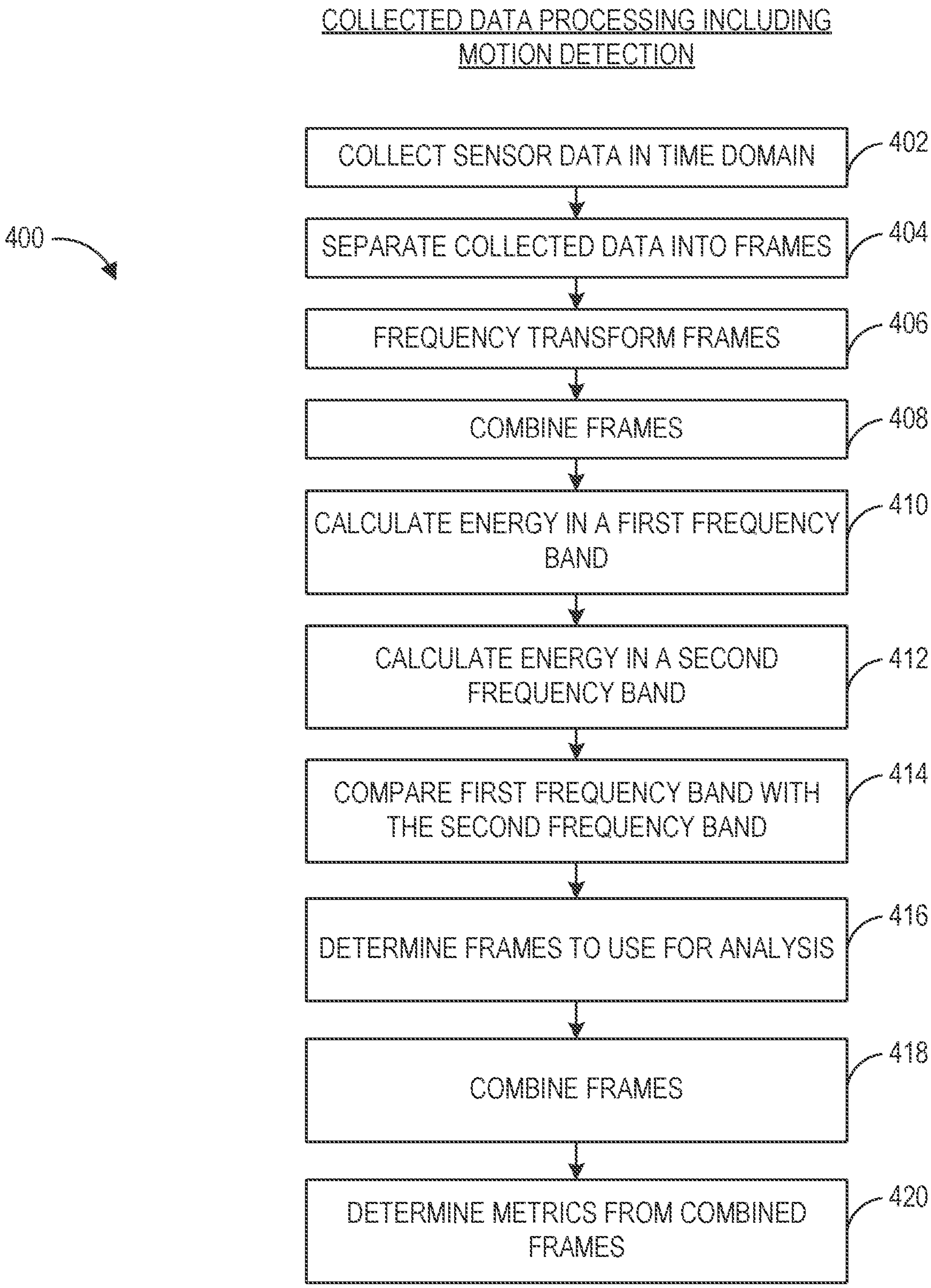
COLLECTED DATA PROCESSING INCLUDING MOTION DETECTION
400
COLLECT SENSOR DATA IN TIME DOMAIN
402
SEPARATE COLLECTED DATA INTO FRAMES
404
FREQUENCY TRANSFORM FRAMES
406
COMBINE FRAMES
408
CALCULATE ENERGY IN A FIRST FREQUENCY BAND
410
CALCULATE ENERGY IN A SECOND FREQUENCY BAND
412
COMPARE FIRST FREQUENCY BAND WITH THE SECOND FREQUENCY BAND
414
DETERMINE FRAMES TO USE FOR ANALYSIS
416
COMBINE FRAMES
418
DETERMINE METRICS FROM COMBINED FRAMES
420

FIG. 4

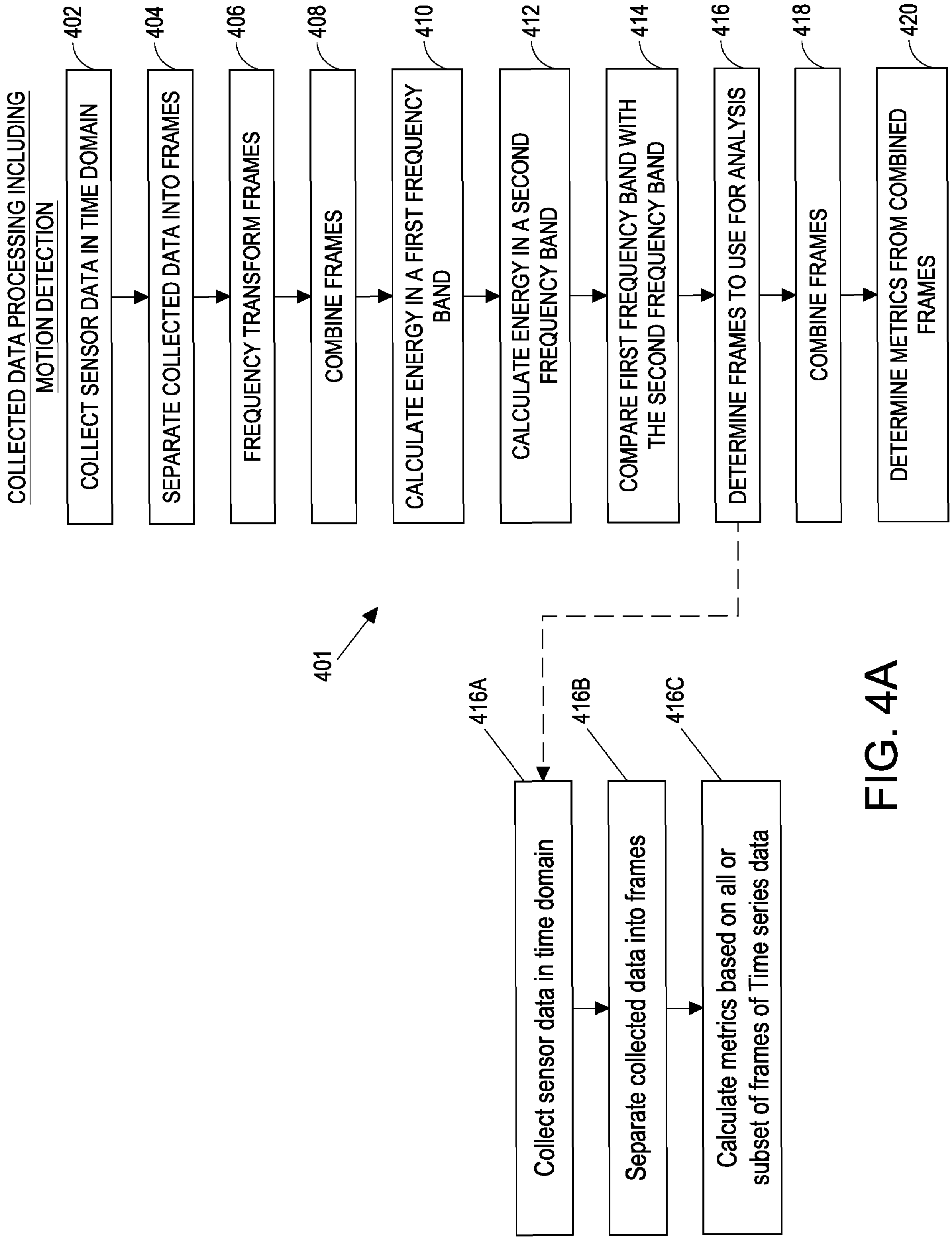
COLLECTED DATA PROCESSING INCLUDING MOTION DETECTION
COLLECT SENSOR DATA IN TIME DOMAIN
402
SEPARATE COLLECTED DATA INTO FRAMES
404
FREQUENCY TRANSFORM FRAMES
406
COMBINE FRAMES
408
CALCULATE ENERGY IN A FIRST FREQUENCY BAND
410
CALCULATE ENERGY IN A SECOND FREQUENCY BAND
412
COMPARE FIRST FREQUENCY BAND WITH THE SECOND FREQUENCY BAND
414
DETERMINE FRAMES TO USE FOR ANALYSIS
416
COMBINE FRAMES
418
DETERMINE METRICS FROM COMBINED FRAMES
420
401
416A
Collect sensor data in time domain
416B
Separate collected data into frames
416C
Calculate metrics based on all or subset of frames of Time series data

FIG. 4A

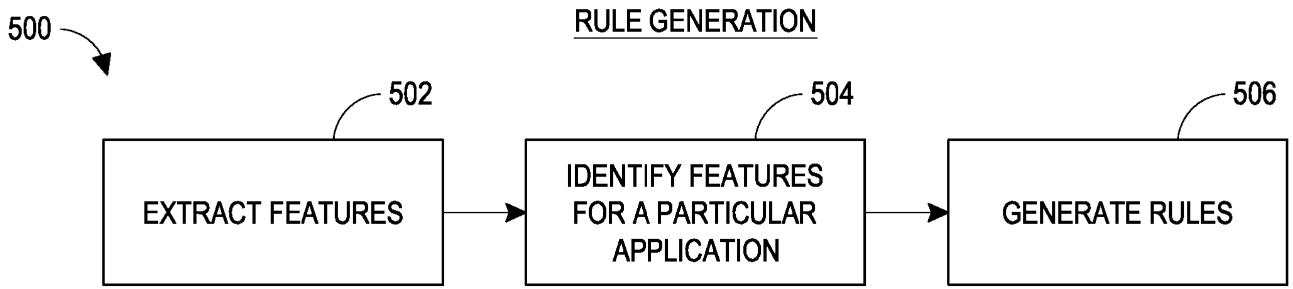
500
RULE GENERATION
502
EXTRACT FEATURES
504
IDENTIFY FEATURES FOR A PARTICULAR APPLICATION
506
GENERATE RULES

FIG. 5A

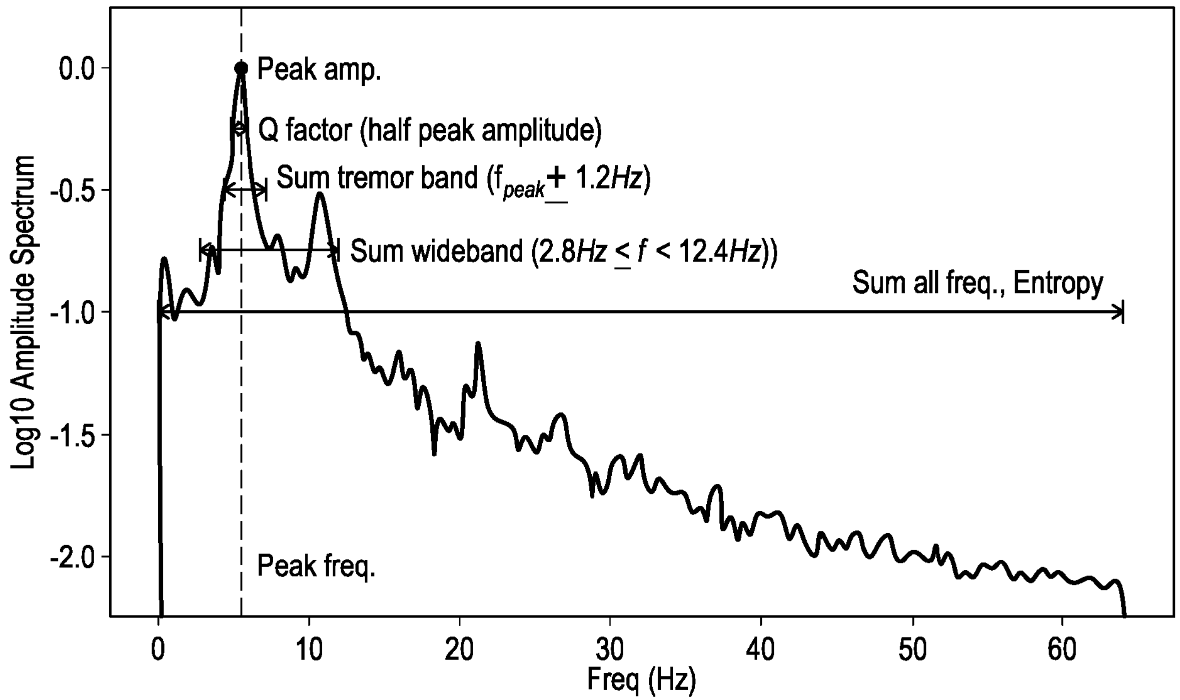
Peak amp.
Q factor (half peak amplitude)
Sum tremor band ($f_{peak} \pm 1.2Hz$)
Sum wideband ($2.8Hz \leq f < 12.4Hz$))
Sum all freq., Entropy
Peak freq.
Log10 Amplitude Spectrum
0.0
-0.5
-1.0
-1.5
-2.0
0
10
20
30
40
50
60
Freq (Hz)

FIG. 5B

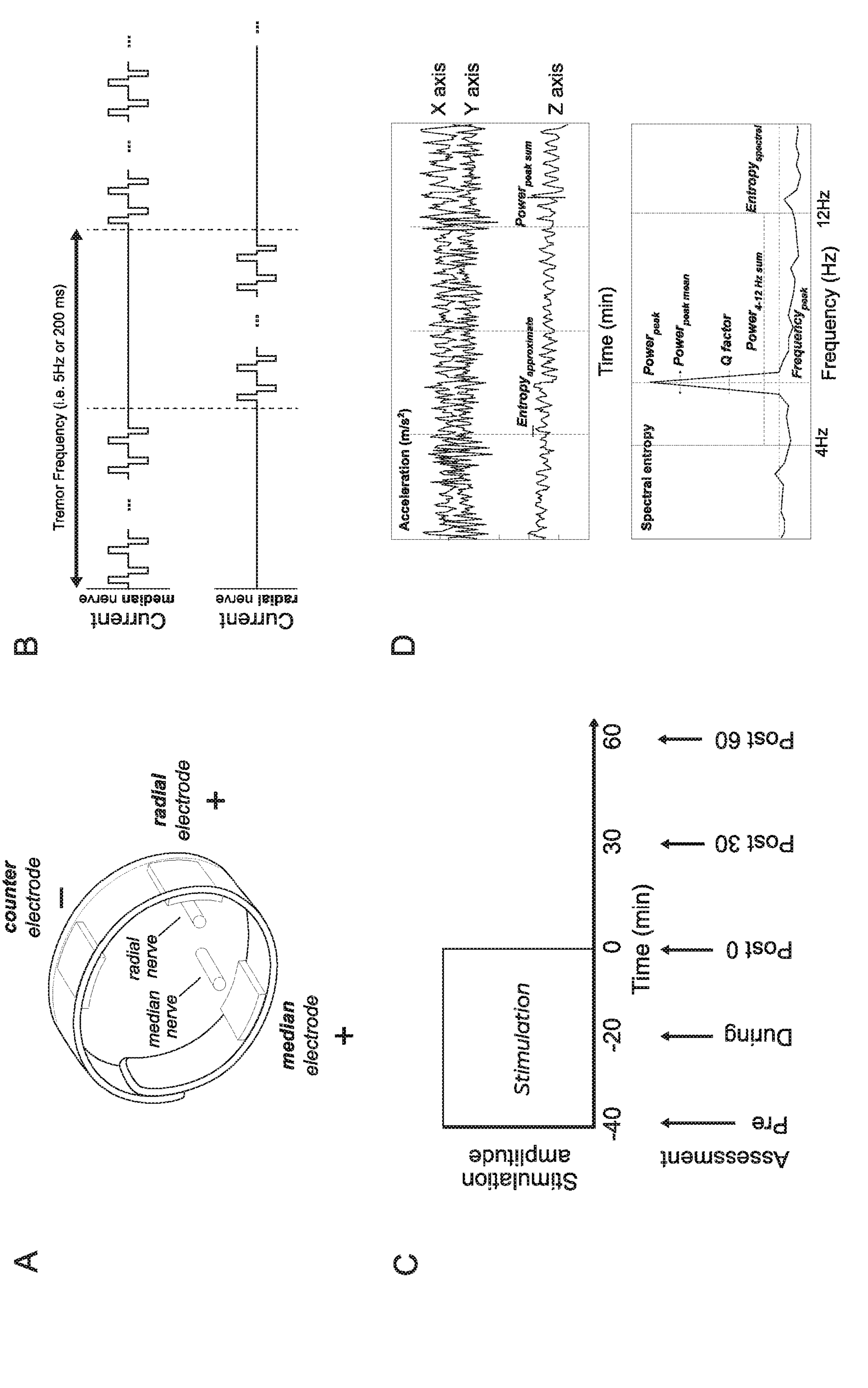
A
counter electrode
–
radial electrode
+
radial nerve
median nerve
median electrode
+
B
Tremor Frequency (i.e. 5Hz or 200 ms)
Current median nerve
Current radial nerve
C
Stimulation amplitude
Stimulation
Time (min)
-40
-20
0
30
60
Assessment
Pre
During
Post 0
Post 30
Post 60
D
Acceleration (m/s²)
X axis
Y axis
Z axis
Entropy approximate
Power peak sum
Time (min)
Spectral entropy
Power peak
Power peak mean
Q factor
Power 4-12 Hz sum
Frequency peak
Entropy spectral
4Hz
12Hz
Frequency (Hz)

FIG. 6

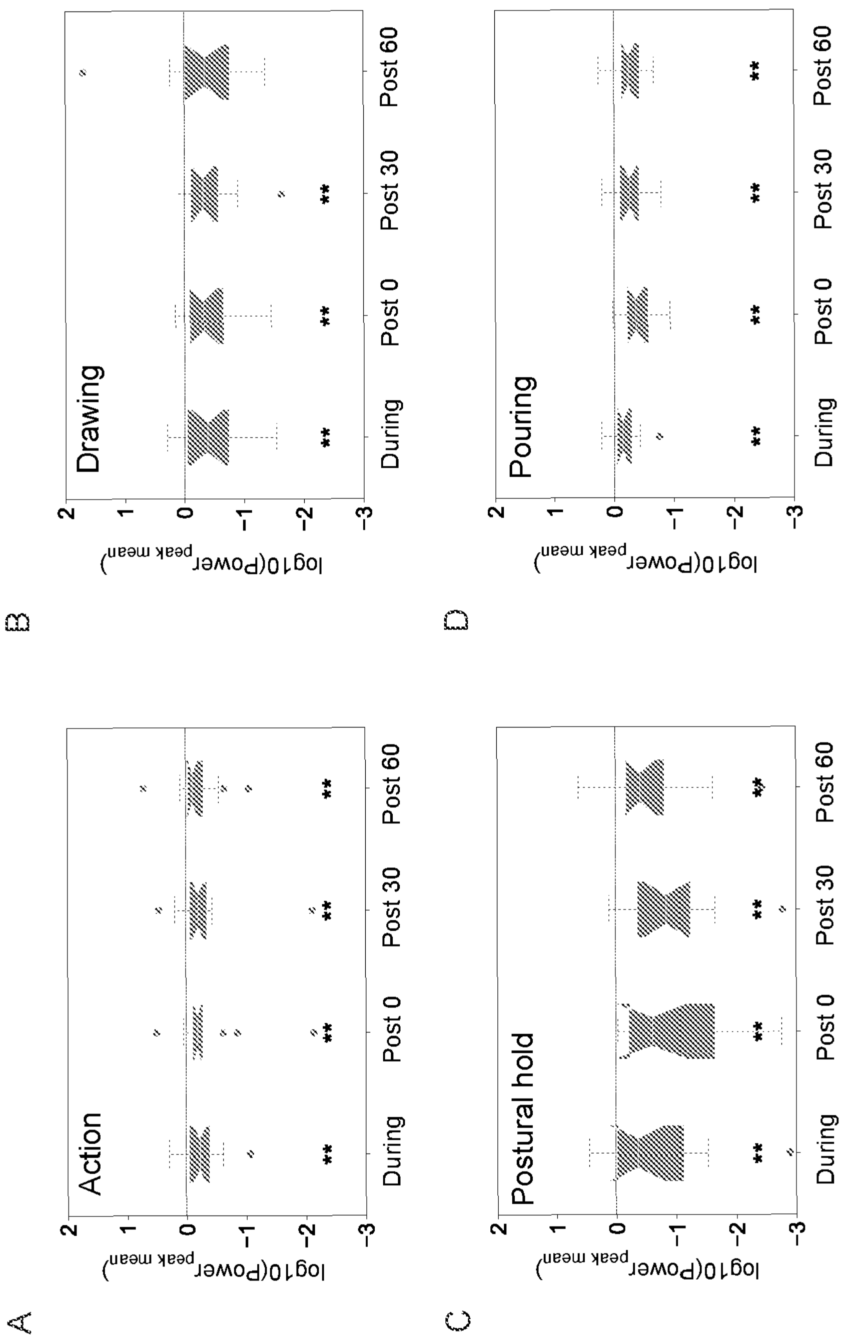
A
Action
B
Drawing
C
Postural hold
D
Pouring
log10(Power peak mean)
During
Post 0
Post 30
Post 60
2
1
0
−1
−2
−3
**

FIG. 7

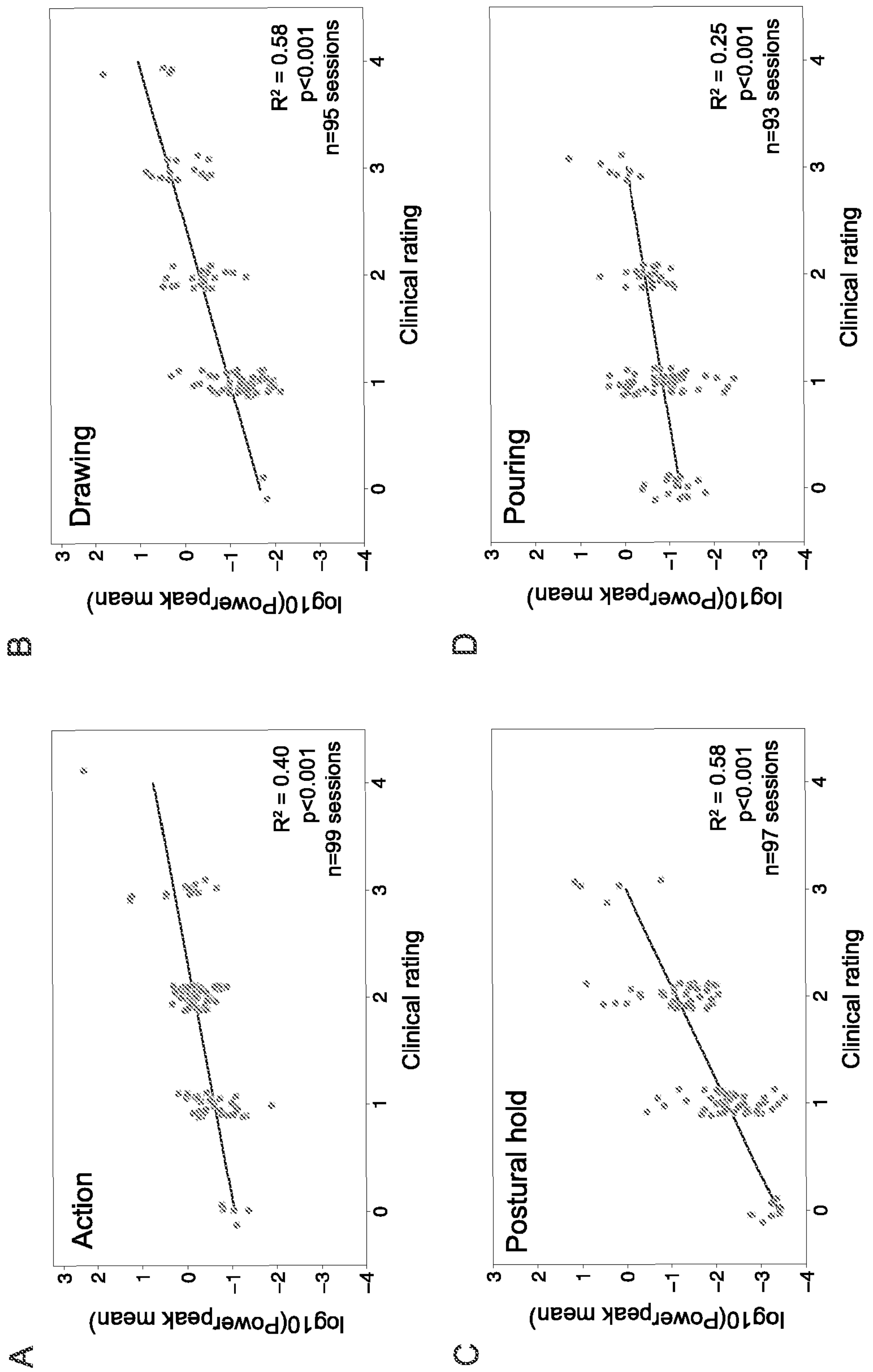
A
Action
log10(Powerpeak mean)
Clinical rating
R² = 0.40
p<0.001
n=99 sessions
B
Drawing
log10(Powerpeak mean)
Clinical rating
R² = 0.58
p<0.001
n=95 sessions
C
Postural hold
log10(Powerpeak mean)
Clinical rating
R² = 0.58
p<0.001
n=97 sessions
D
Pouring
log10(Powerpeak mean)
Clinical rating
R² = 0.25
p<0.001
n=93 sessions

FIG. 8

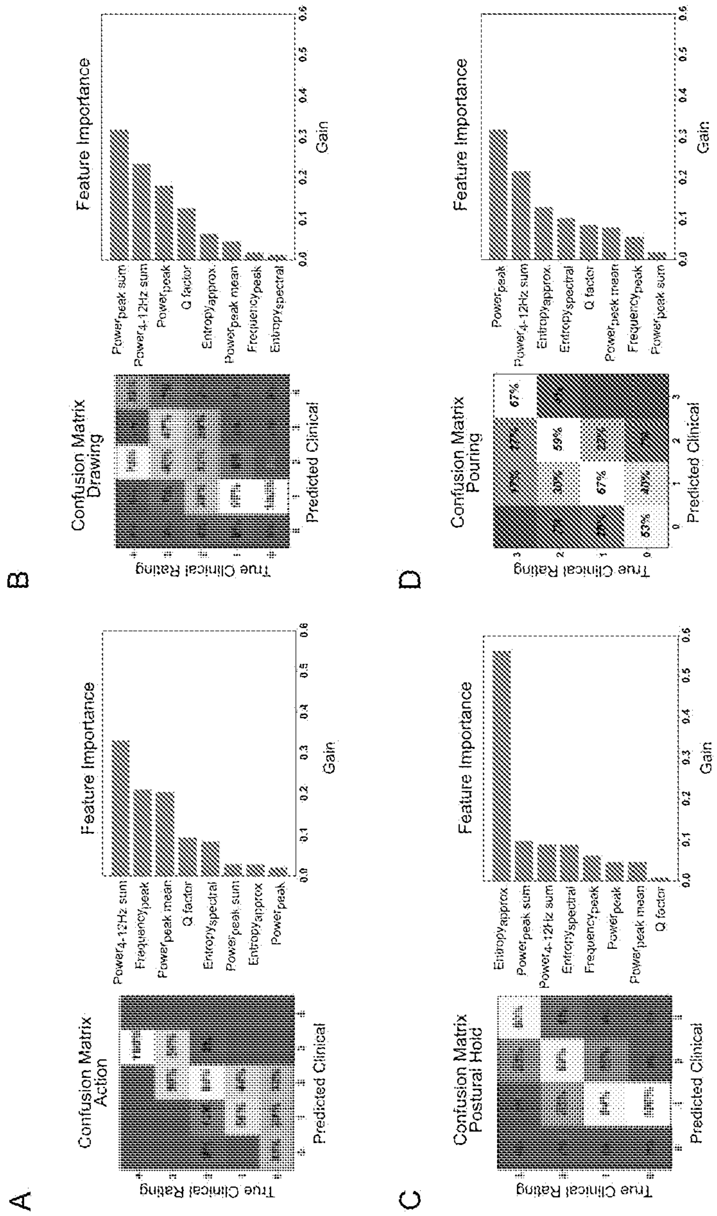
A
Confusion Matrix
Action
True Clinical Rating
Predicted Clinical
Feature Importance
Gain
B
Confusion Matrix
Drawing
True Clinical Rating
Predicted Clinical
Feature Importance
Gain
C
Confusion Matrix
Postural Hold
True Clinical Rating
Predicted Clinical
Feature Importance
Gain
D
Confusion Matrix
Pouring
True Clinical Rating
Predicted Clinical
Feature Importance
Gain

FIG. 9

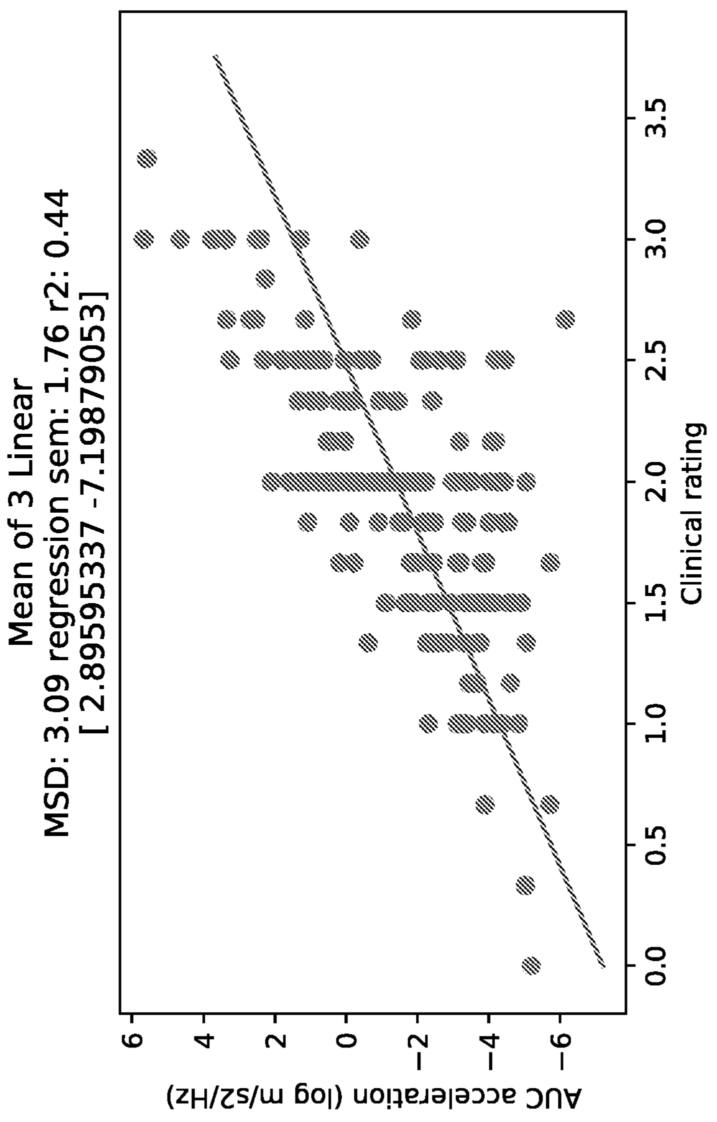
Mean of 3 Linear
MSD: 3.09 regression sem: 1.76 r2: 0.44
[ 2.89595337 -7.19879053]
AUC acceleration (log m/s2/Hz)
6
4
2
0
−2
−4
−6
0.0
0.5
1.0
1.5
2.0
2.5
3.0
3.5
Clinical rating

FIG. 10

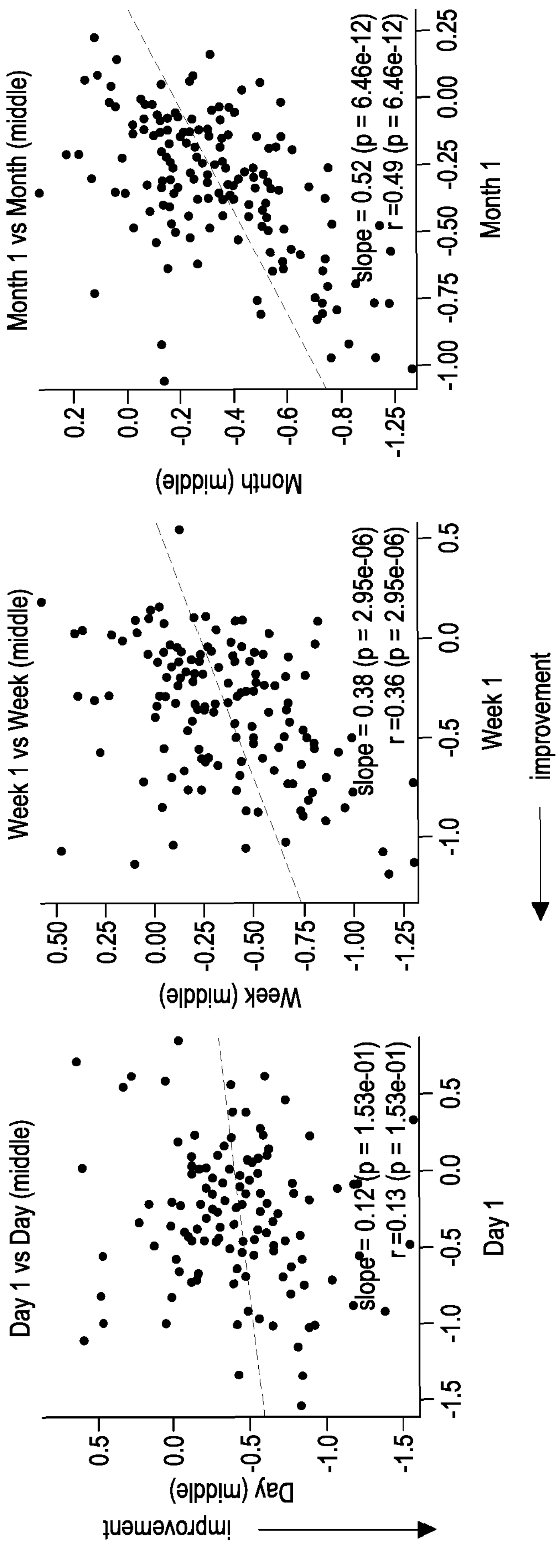
Day 1 vs Day (middle)
slope = 0.12 (p = 1.53e-01)
r =0.13 (p = 1.53e-01)
Day 1
Day (middle)
improvement
Week 1 vs Week (middle)
slope = 0.38 (p = 2.95e-06)
r =0.36 (p = 2.95e-06)
Week 1
Week (middle)
improvement
Month 1 vs Month (middle)
slope = 0.52 (p = 6.46e-12)
r =0.49 (p = 6.46e-12)
Month 1
Month (middle)

FIG. 11

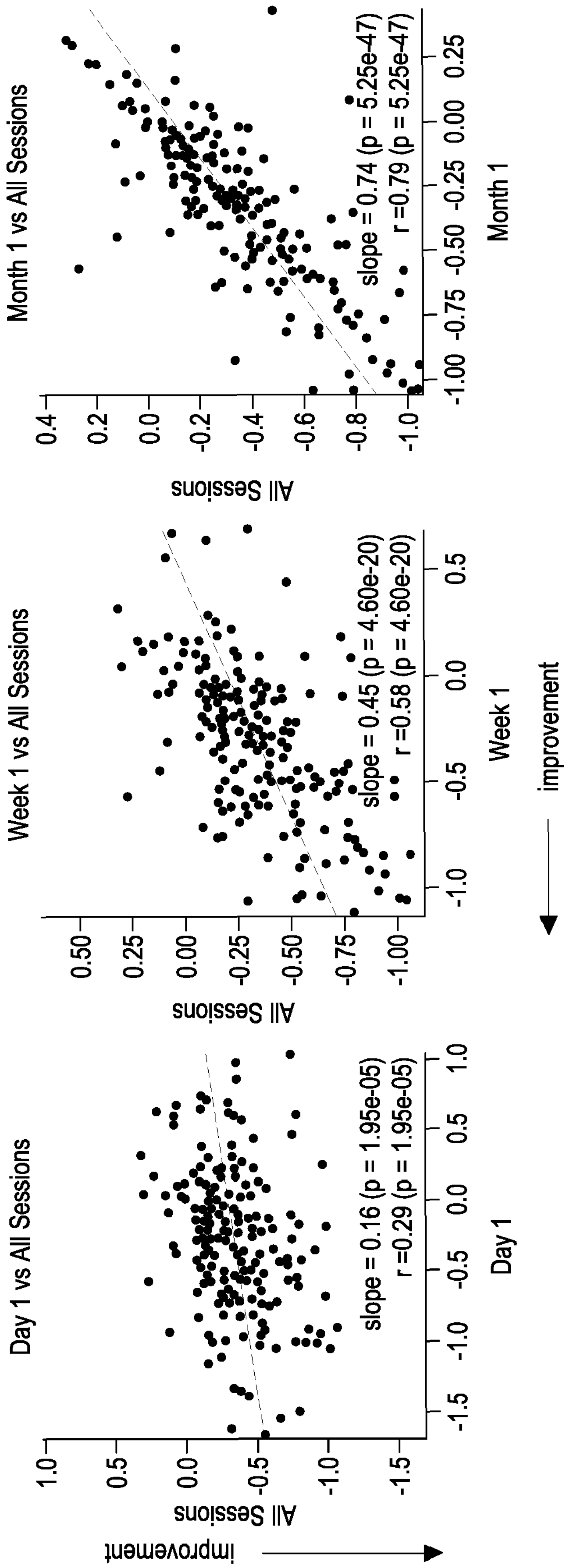

Day 1 vs All Sessions
slope = 0.16 (p = 1.95e-05)
r =0.29 (p = 1.95e-05)
Day 1
All Sessions
improvement
Week 1 vs All Sessions
slope = 0.45 (p = 4.60e-20)
r =0.58 (p = 4.60e-20)
Week 1
All Sessions
improvement
Month 1 vs All Sessions
slope = 0.74 (p = 5.25e-47)
r =0.79 (p = 5.25e-47)
Month 1
All Sessions


FIG. 12

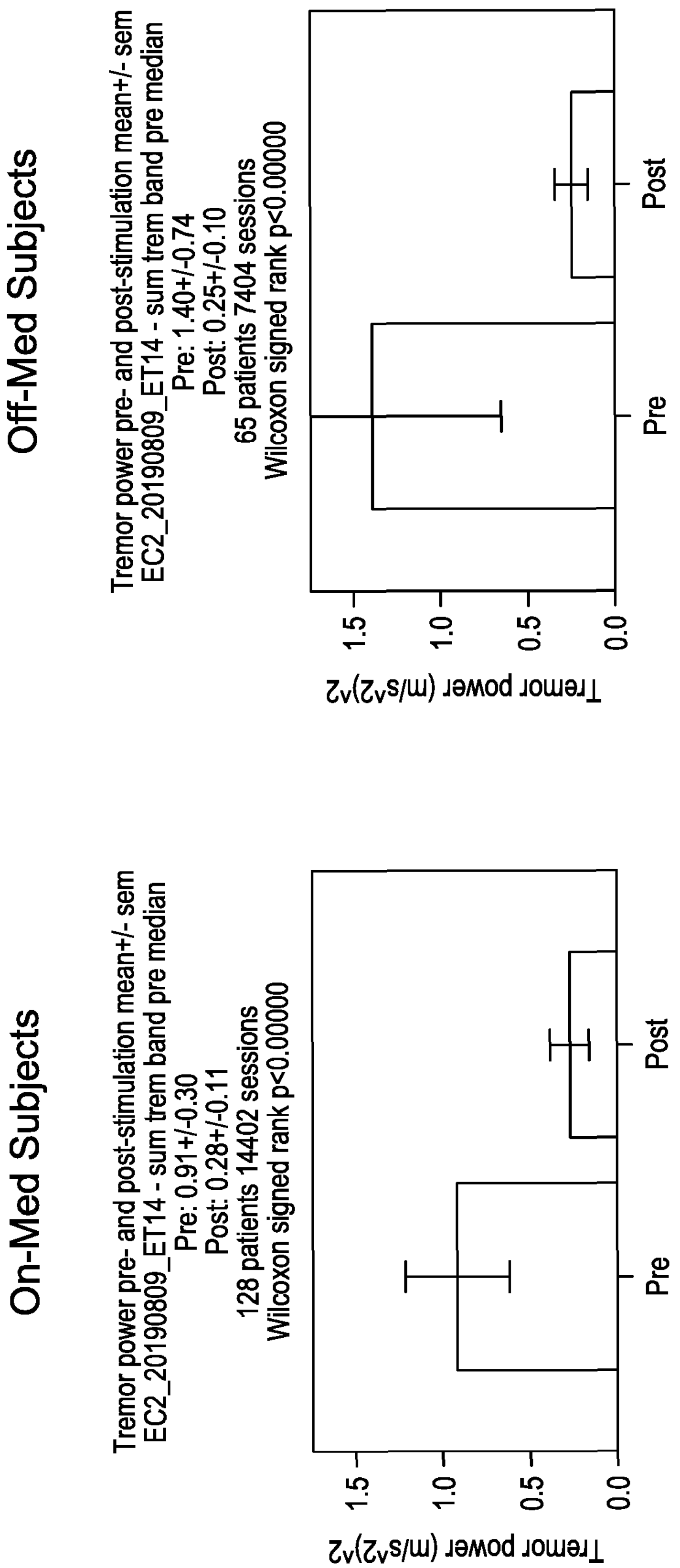
On-Med Subjects
Tremor power pre- and post-stimulation mean+/- sem
EC2_20190809_ET14 - sum trem band pre median
Pre: 0.91+/-0.30
Post: 0.28+/-0.11
128 patients 14402 sessions
Wilcoxon signed rank p<0.00000
Tremor power (m/s^2)^2
1.5
1.0
0.5
0.0
Pre
Post
Off-Med Subjects
Tremor power pre- and post-stimulation mean+/- sem
EC2_20190809_ET14 - sum trem band pre median
Pre: 1.40+/-0.74
Post: 0.25+/-0.10
65 patients 7404 sessions
Wilcoxon signed rank p<0.00000
Tremor power (m/s^2)^2
1.5
1.0
0.5
0.0
Pre
Post

FIG. 13

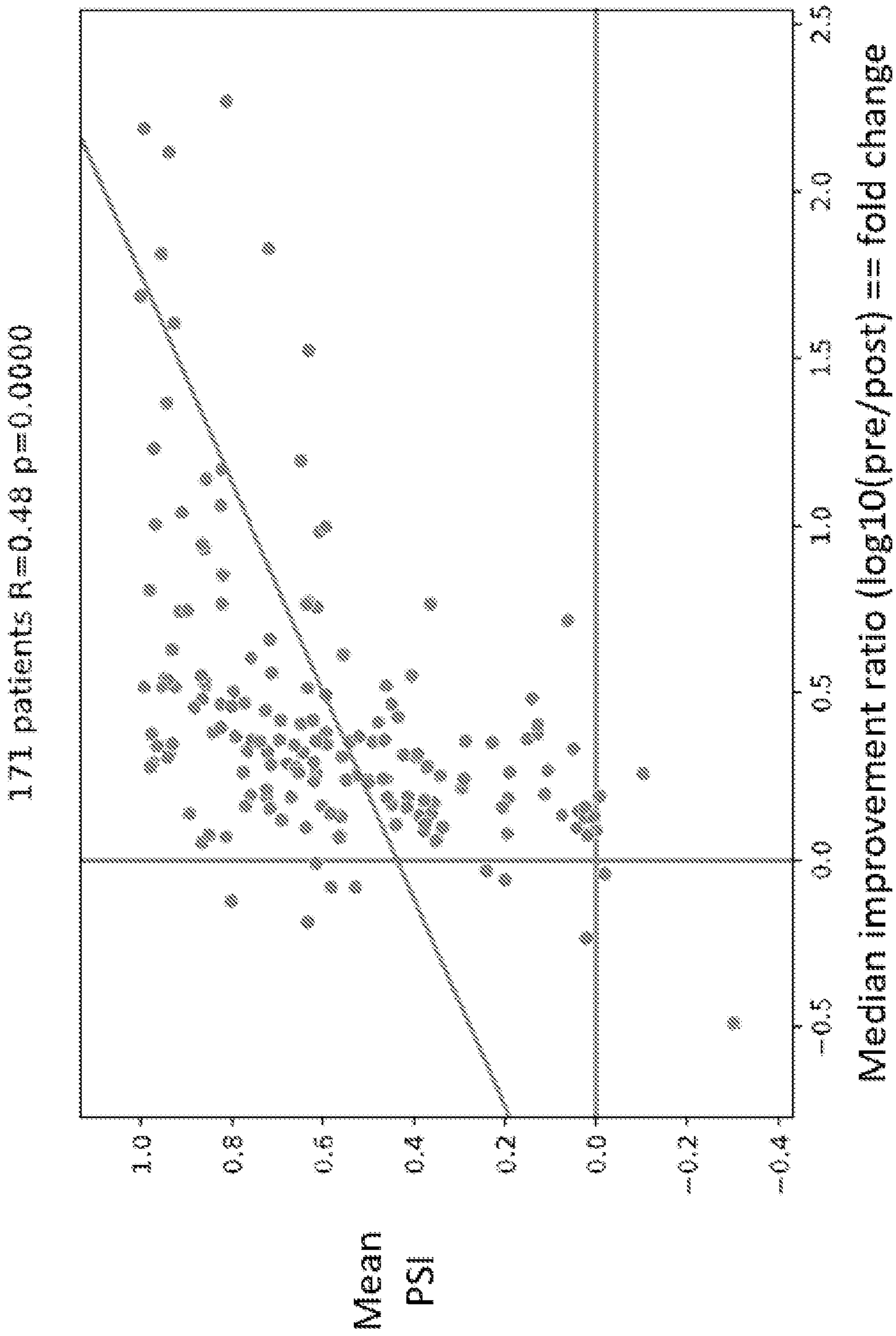
171 patients R=0.48 p=0.0000
Mean
PSI
1.0
0.8
0.6
0.4
0.2
0.0
−0.2
−0.4
−0.5
0.0
0.5
1.0
1.5
2.0
2.5
Median improvement ratio (log10(pre/post) == fold change

FIG. 14

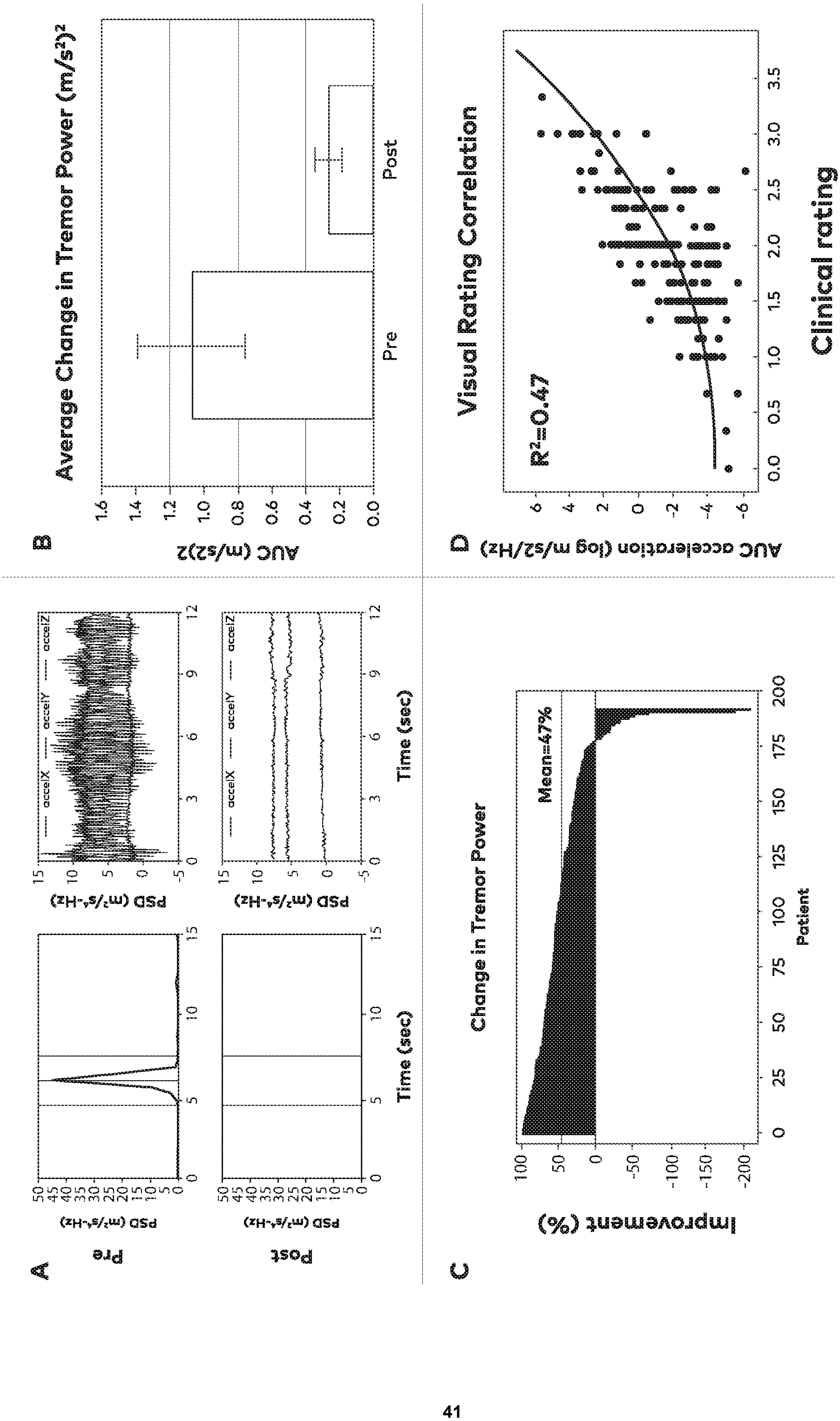
A
Pre
Post
PSD (m²/s⁴-Hz)
Time (sec)
accelX
accelY
accelZ
B
Average Change in Tremor Power (m/s²)²
AUC (m/s2)2
Pre
Post
C
Change in Tremor Power
Improvement (%)
Mean=47%
Patient
D
Visual Rating Correlation
R²=0.47
AUC acceleration (log m/s2/Hz)
Clinical rating

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017132067 A
- WO 2014089549 A
- US 2014336722 A
- WO 2018028170 A
- US 2018264263 A
- US 2016198998 A
- US 9452287 B, Rosenbluth
- US 9802041 B, Wong
- WO 2016201366 A, Wong
- WO 2017023864 A, Hamner
- WO 2017053847 A, Hamner
- WO 2018009680 A, Wong
- WO 2018039458 A, Rosenbluth